# FASCICULE DE BREVET EUROPEEN

(11) **EP 1 218 366 B1**
(45) Date de publication et mention de la délivrance du brevet: **14.09.2005**
(21) Numéro de dépôt: 00967956.4
(22) Date de dépôt: 05.10.2000
(51) Int. Cl.: C07D 307/60, C07C 323/19

(54) **PROCEDE DE PREPARATION D'INHIBITEURS COX-2**
VERFAHREN ZUR HERSTELLUNG VON COX-2 INHIBITOREN
METHOD FOR PRODUCING COX-2 INHIBITORS

(30) Priorité: 08.10.1999 FR 9912583
(43) Date de publication de la demande: 03.07.2002
(73) Titulaire: MERIAL, 69002 Lyon (FR)
(72) Inventeur: CANALI, Laetitia, F-69560 Sainte Colombe (FR); CRUCIANI, Paul, F-69740 Genas (FR); ODDON, Gilles, 69003 Lyon (FR)
(74) Mandataire: Nargolwalla, Cyra
(86) Numéro de dépôt international: PCT/FR2000/002770
(87) Numéro de publication internationale: WO 2001/027098

(56) Documents cités:
- WO-A-97/14691
- WO-A-97/45420
- WO-A-98/41516
- W. ADAM ET AL.: "Epoxidation of Enol Silyl Ethers, Phosphates, Esters, and Lactones by Dimethyldioxirane" CHEMISCHE BERICHTE, vol. 124, 1991, pages 2361-2368, XP002145830 WEINHEIM DE
- C. L. STEVENS ET AL.: "Epoxyethers. IV. Mechanism of the Opening of an Epoxyether with an Organic Acid." JOURNAL OF THE AMERICAN CHEMICAL SOCIETY, vol. 75, 1953, pages 5975-5978, XP002145831 DC US
- MURRAY R W ET AL: "DIOXIRANES: SYNTHESIS AND REACTIONS OF METHYLDIOXIRANES" JOURNAL OF ORGANIC CHEMISTRY,US,AMERICAN CHEMICAL SOCIETY. EASTON, vol. 50, 1985, pages 2847-2853, XP002045042 ISSN: 0022-3263
- T. KITAMURA ET AL.: "Ipso Substitution of Triarylvinyl Cations by Alkoxide Anions" JOURNAL OF THE AMERICAN CHEMICAL SOCIETY, vol. 113, no. 19, 1991, pages 7255-7261, XP002145832 DC US

## Description

L'invention concerne un procédé de préparation de (4-alkylsulfonyl)-phényl-2-(5H)-furanones, qui sont des composés inhibiteurs de la cyclooxygénase-2 (COX-2) ; ainsi que des composés intermédiaires nouveaux utiles pour la préparation de ces composés.

Des composés (4-alkylsulfonyl)-phenyl-2-(5H)-furanones utiles comme inhibiteurs COX-2 ainsi que leurs applications pharmacologiques comme anti-inflammatoires sont connus et décrits dans les documents suivants WO 97/44027, WO 97/28121, WO 98/41516, WO 96/19469, WO 97/16435 et WO 97/14691.

La synthèse de ces composés fait intervenir un procédé en plusieurs étapes faisant intervenir un intermédiaire de type 4-alkylsulfonyl-α-bromoisobutyrophénone.

Ainsi WO 97/45420 décrit un procédé de préparation de (méthyl-4-sulfonyl)-phényl-2-(5H)-furanones à partir du thioanisole faisant intervenir cinq étapes.

La seconde étape de ce procédé consiste à bromer la 4-thiométhyl-isobutyrophénone pour obtenir de la 4-thiométhyl-α-bromoisobutyrophénone.

Dans l'étape suivante la 4-thiométhyl-α-bromoisobutyrophénone est oxydée en 4-méthylsulfonyl-α-bromoisobutyrophénone qui est un composé hautement allergisant, ce composé est ensuite estérifié avec un acide carboxylique pour former un ester de 2-méthyl-1-(4'-méthylsulfonylphényl)-1-oxo-prop-2-yle.

Cette réaction s'accompagne par ailleurs d'un certain nombre de sous-produits parmi lesquels un produit d'élimination, la 4-(4'-méthylsulfonylphényl)-2-méthyl-propènone.

Le but de l'invention est de proposer une alternative au procédé décrit dans WO 97/45420 et notamment un procédé général de préparation de composés (4-alkylsulfonylphényl)-2-(5H)-furanones substituées évitant le problème posé par l'intermédiaire de type α-bromoisobutyrophénone, qui soit facile à mettre en oeuvre, évite la formation du sous-produit d'élimination et fournisse un rendement acceptable en produit final.

Les travaux réalisés par les inventeurs ont à présent permis de proposer un procédé répondant à ces attentes, et qui permet notamment d'éviter le passage par un dérivé bromosulfone toxique et la formation du sous-produit précité.

L'invention a ainsi pour objet un procédé de préparation de composés de formule générale I : 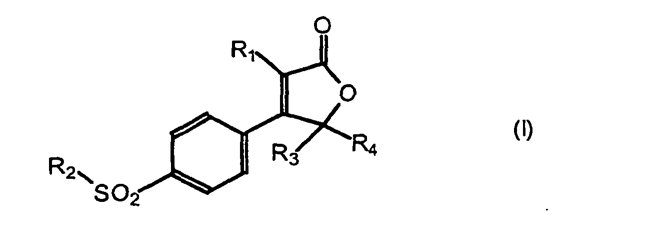 dans laquelle
- R₁ est choisi parmi les groupes
   (a) OR₅ où R₅ représente un groupe choisi parmi
      (1) un groupe C₁-C₆ alkyle linéaire ramifié ou cyclique ;
      (2) un groupe phényle ou naphtyle mono-, di- ou tri-substitué
         dans lesquels les substituants sont choisis parmi :
         - hydrogène ;
         - halogène ;
         - (C₁-C₃) alkoxy ;
         - CN;
         - (C₁-C₃) fluoroalkyle ;
         - (C₁-C₃)alkyle ;
         - -COOH ;
         et
   (b) phényle mono-, di- ou tri-substitué dans lequel les substituants sont choisis parmi :
      - hydrogène ;
      - halogène ;
      - (C₁-C₃) alkoxy ;
      - CN;
      - (C₁-C₃) fluoroalkyle ;
      - (C₁-C₃)alkyle ;
      - -COOH;
- R₂ représente un groupe (C₁-C₆)alkyle ;
- R₃ et R₄ représentent indépendamment l'un de l'autre un atome d'hydrogène ou un groupe CHR₆R₇
   dans lequel R₆ et R₇ sont indépendamment l'un de l'autre choisis parmi :
   - hydrogène ;
   - (C₁-C₁₀)alkyle ;
   - (C₁-C₁₀) alkoxy ;
   - OH ;
   - CN ;
   - CH₂CN ;
   - OCOR₈ ;
   - (C₁-C₆)fluoroalkyle ;
   - halogène ;
   - CON(R₈)₂ ;
   - phényle mono-, di- ou tri-substitué ;
   - hétéroaryle mono-, di- ou tri-substitué ;
   les substituants étant choisis parmi :
   - hydrogène ;
   - halogène ;
   - (C₁-C₆)alkyle ;
   - (C₁-C₁₀)alkoxy ;
   - CN ;
   - CF₃ ;
   - N₃ ;
   - C(R₉)(R₁₀)-OH ;
   - C(R₉) (R₁₀)-O-(C₁-C₄)alkyle ;
   - (C₁-C₆)fluoroalkyle ;
- R₈ est choisi parmi :
   - hydrogène ;
   - (C₁-C₆)alkyle ;
   - phényle mono-, di- ou trisubstitué, les substituants étant choisis parmi hydrogène, halogène, (C₁-C₆)alkyle, (C₁-C₆)alkoxy, (C₁-C₆)alkylthio, CN ou CF₃; et
   - benzyle mono-, di- ou trisubstitué, les substituants étant choisis parmi hydrogène, halogène, (C₁-C₆)alkyle, (C₁-C₆)alkoxy, (C₁-C₆)alkylthio, CN ou CF₃ ;
- ou deux groupes R₈ forment ensemble avec l'atome d'azote auxquels ils sont attachés un cycle ayant de 5 à 7 atomes, et comprenant éventuellement un hétéroatome choisi parmi O, S ou NR₉ ;
- R₉ et R₁₀ sont indépendamment l'un de l'autre choisis parmi :
   - hydrogène ; et
   - (C₁-C₁₀)alkyle; ou
   forment ensemble avec l'atome auquel ils sont attachés un cycle ayant de 3 à 7 atomes de carbone et le cas échéant un atome d'azote ;
   caractérisé en ce qu'il comprend les étapes suivantes :
   a) réaction d'un composé de formule générale II : 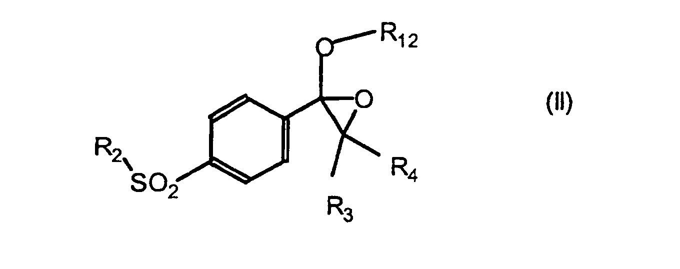 dans laquelle R₂, R₃ et R₄ sont tels que définis précédemment et R₁₂ représente un groupe alkyle en C₁-C₆,
      avec un acide de formule générale III : 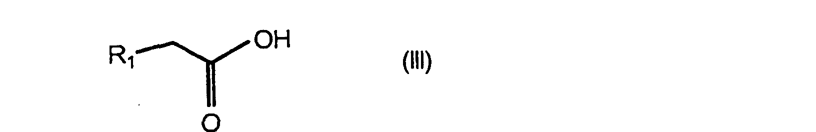 dans laquelle R₁ est tel que défini précédemment, en milieu anhydre, pour former un composé de formule IV : 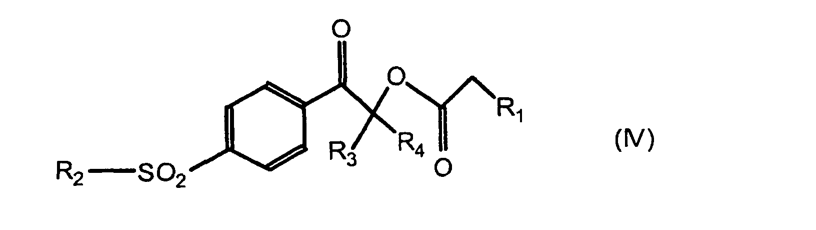 R₁,R₂, R₃ et R₄ étant tels que définis ci-dessus;
   b) réaction du composé de formule IV avec une base forte dans un solvant aprotique pour obtenir un composé cyclique intermédiaire de formule V : 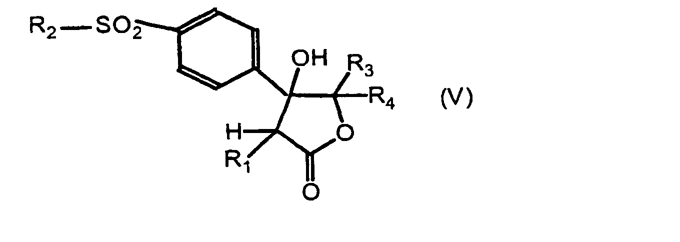 qui, après élimination d'une molécule d'eau forme un composé de formule générale I ; et
   c) isolement du composé de formule générale I ainsi obtenu.

   La réaction de l'étape a) a lieu dans un solvant anhydre, de préférence un éther, par exemple le diéthyléther, ou le méthyltertiobutyléther. La température de réaction est avantageusement comprise entre -20 et 40°C. A la fin de l'étape a), on obtient un composé de formule générale IV ainsi que des produits secondaires en quantité mineure. Toutefois, il ne se forme pas de produit d'élimination précité.
   Pour la réaction de l'étape b), la base forte est choisie avantageusement parmi le 1,8-diazabicyclo[5.4.0]undec -7-ène (DBU), le 1,4-diazabicyclo[2.2.2]octane (DABCO) et le 1,5-diazabicyclo[4.3.0]non-5-ène (DBN).
   L'élimination d'une molécule d'eau est réalisée d'une manière connue en soi, avantageusement par déshydratation thermique en présence d'un agent déshydratant.
   L'agent déshydratant peut être choisi notamment parmi les esters d'acides trifluoroacétiques, par exemple le trifluoroacétate d'isopropyle, les esters d'acides trichloracétiques et les esters d'acides alkyl ou arylsulfoniques.
   La réaction a lieu de préférence dans un solvant aprotique tel que l'acétonitrile, le N,N-diméthylformamide, le N-méthylsulfoxyde, le proprionitrile et le nitrométhane.
   La déshydratation est réalisée par chauffage au reflux.
   Le rapport molaire de l'ester de formule IV à la base forte se situe généralement entre 1:1 et 1:2, un rapport de 1:1,5 étant préféré.
   Le rapport molaire de l'ester cyclique de formule V à l'agent déshydratant est généralement de 1:1 à 1:2, un rapport de 1:1,2 étant préféré.
   La réaction est effectuée à une température se situant de préférence entre 0°C et la température de reflux du solvant.
   Des conditions de réaction particulièrement avantageuses sont réalisées par utilisation d'un mélange de 1,2 équivalents de trifluoroacétate d'isopropyle et de 1,5 équivalents de DBU dans l'acétonitrile au reflux. Dans ces conditions, la réaction est terminée après 24 heures et le produit cristallise par addition d'eau après élimination partielle de l'acétonitrile. Pour plus de précisions, on se rapportera à la description de la demande de brevet WO 97/45420.
   L'étape c) est réalisée de manière connue en soi, notamment par élimination du solvant, précipitation du produit, recristallisation, etc...
   Le composé époxy de formule générale II peut être obtenu par réaction d'un composé de formule générale VI : 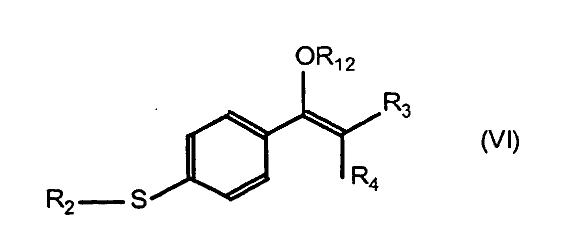 dans laquelle R₂, R₃, R₄ et R₁₂ sont tels que définis précédemment, avec un agent oxydant.
   A titre d'agent oxydant, on peut citer notamment les peracides organiques, tels que l'acide méta-chloroperbenzdïque et l'acide peracétique ou les dioxiranes tels que le diméthyldioxirane, généré ou non *in situ.* La température de réaction est avantageusement comprise entre -40°C et 30°C.
   L'agent oxydant est utilisé en excès par rapport au composé de formule générale II (3 à 40 équivalents), de manière à oxyder d'une part la fonction oléfinique en époxyde, d'autre part la fonction sulfure en sulfone.
   Le composé de formule générale VI peut être obtenu par réaction d'un composé de formule générale VII : 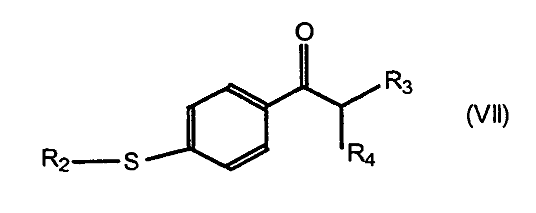 dans laquelle R₂, R₃ et R₄ sont tels que définis précédemment, avec un alcool de formule générale VIII :

   HO R₁₂ (VIII)

   R₁₂ étant tel que défini ci-dessus, en présence d'une quantité catalytique d'acide et d'un agent déshydratant.
   Avantageusement, l'acide est choisi parmi les acides sulfoniques, par exemple l'acide p-toluène sulfonique ou les acides minéraux, par exemple l'acide chlorhydrique. A titre d'agent déshydratant, on préfère les orthoformiates d'alkyle en C₁-C₆.
   La réaction est réalisée dans un excès d'alcool de formule générale VIII, celui-ci servant de solvant réactif.
   Dans le composé de formule générale VIII, R₁₂ est avantageusement un groupe méthyle, l'alcool étant le méthanol.
   L'invention a également pour objet un procédé de préparation d'un composé de formule générale I tel que défini précédemment, caractérisé en ce qu'il comprend les étapes suivantes :
   (1) réaction d'un composé de formule générale IX : 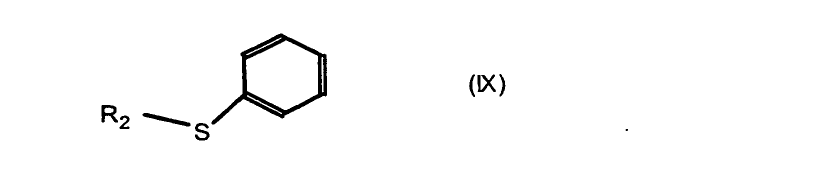 dans laquelle R₂ est tel que défini précédemment, dans un solvant inerte en présence d'un acide de Lewis avec un composé de formule générale X : 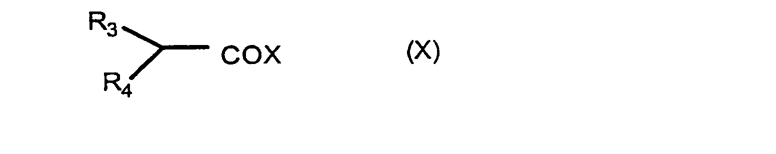 dans laquelle X est un groupe partant, de préférence un atome de chlore pour former un composé de formule générale VII : 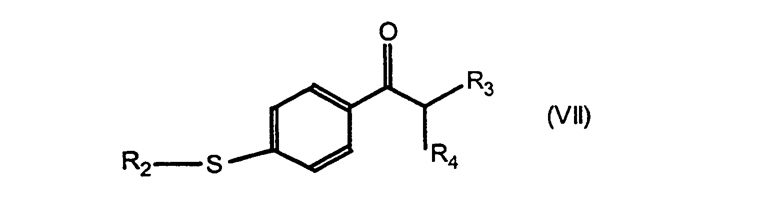 dans laquelle R₂, R₃ et R₄ sont tels que définis précédemment,
   (2) réaction du composé de formule générale VII avec un alcool de formule générale VIII :

      R₁₂-OH (VIII)

      dans laquelle R₁₂ représente un groupe (C₁-C₆)alkyle pour former un composé de formule générale VI : 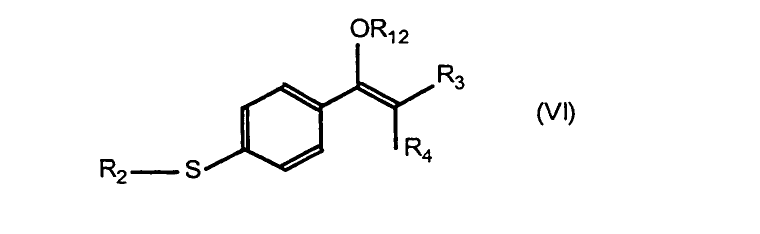 dans laquelle R₂, R₃, R₄ et R₁₂ sont tels que définis précédemment,
   (3) réaction du composé de formule générale VI avec un agent oxydant pour obtenir un composé de formule générale II : 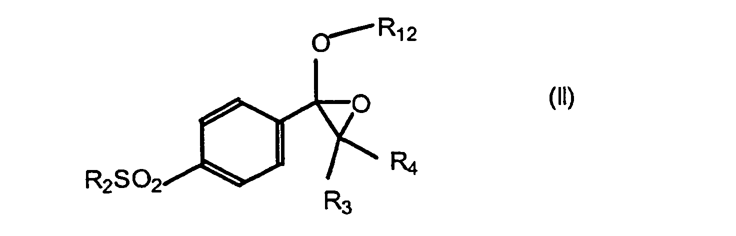 dans laquelle R₂, R₃, R₄ et R₁₂ sont tels que définis précédemment ;
   (4) réaction du composé de formule générale II telle que définie à l'étape (3)
      avec un acide de formule générale III : 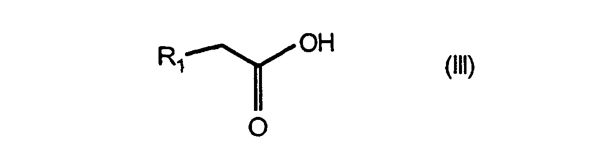 dans laquelle R₁ est tel que défini précédemment, en milieu anhydre, pour former un composé de formule IV : 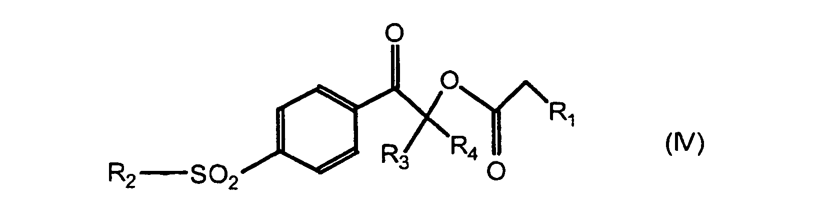 R₁, R₂, R₃ et R₄ étant tels que définis ci-dessus;
   (5) réaction du composé de formule IV avec une base forte dans un solvant aprotique pour obtenir un composé cyclique intermédiaire de formule V : 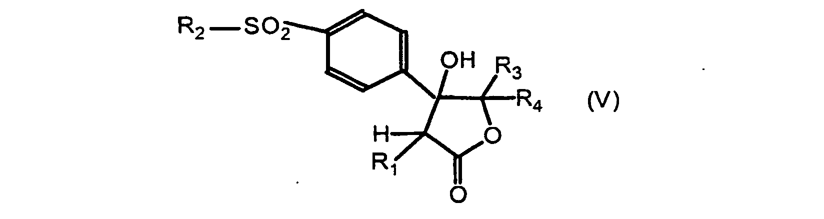 qui, après élimination d'une molécule d'eau forme un composé de formule générale 1 ; et
   (6) isolement du composé de formule générale I ainsi obtenu.

Pour la réaction de l'étape (1), l'acide de Lewis est avantageusement choisi parmi AlCl₃, FeCl₃, TiCl₄ et SnCl₄ sans toutefois être limité à ceux-ci. Les solvants non réactifs comprennent les hydrocarbures halogénés et polyhalogénés tels que les mono- ou dihalo(C₁-C₄)alkyles, par exemple le dichlorométhane ; les solvants aromatiques tels que le nitrobenzène ou des composés aromatiques halogénés, ainsi que des hydrocarbures linéaires ramifiés ou cycliques en C₆-C₁₀ comprenant notamment l'hexane, le cyclohexane, le méthylcyclohexane ou le CS₂. Pour cette étape, on peut en particulier choisir le cyclohexane ou le dichlorobenzène. Le rapport molaire du composé de formule générale IX au composé de formule générale X est généralement compris entre 1:1,5 et 1,5:1, un rapport de 1:1 à 1:1,5 étant préféré. La réaction est généralement effectuée avec un excès du composé de formule générale X. Généralement, le rapport molaire du composé de formule générale IX à l'acide de Lewis est compris entre 1:1,5 et 1,5:1. De préférence, le rapport molaire du composé de formule générale IX à l'acide de Lewis est compris entre 1:1 et 1:1,5. La réaction peut être avantageusement réalisée dans une gamme de température comprise entre 0 et 25°C, de préférence 5 et 15°C. Les réactifs sont mis à réagir jusqu'à achèvement de la réaction, lequel se produit après un intervalle de temps allant de 8 à 4 heures, généralement de 1 à 2 heures. La réaction est effectuée de préférence sous atmosphère d'azote. Les étapes (2) à (6) sont effectuées dans des conditions telles que décrites précédemment.

Les composés de formule générale IX et X sont des composés commercialisés ou pouvant être facilement préparés par l'homme du métier en mettant en oeuvre des procédés de routine bien connus.

Dans un premier mode de réalisation du procédé de l'invention R₁ est un groupe RO, R étant tel que défini précédemment pour R₅.

Le composé de formule générale I devient alors un composé de formule générale la :

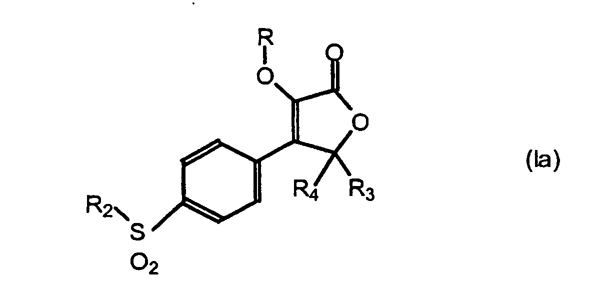

Le procédé selon l'invention comprend dans ce cas les étapes suivantes :
a) réaction d'un composé de formule générale II 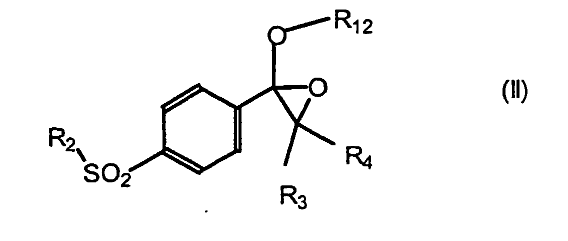 dans laquelle R₂, R₃ et R₄ sont tels que définis précédemment et R₁₂ représente un groupe alkyle en C₁-C₆,
   avec un acide de formule générale IIIa 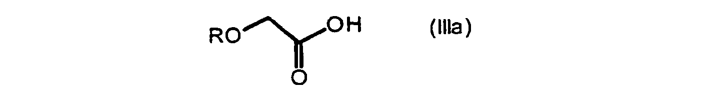 dans laquelle R est tel que défini ci-dessus, en milieu anhydre pour former un composé de formule générale IVa : 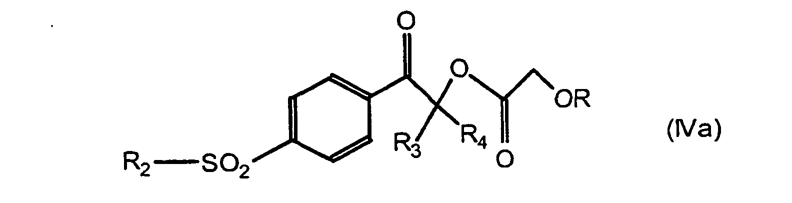 dans laquelle R, R₂, R₃ et R₄ sont tels que définis précédemment.
b) réaction du composé de formule IVa avec une base forte dans un solvant aprotique pour obtenir un composé cyclique intermédiaire de formule Va : 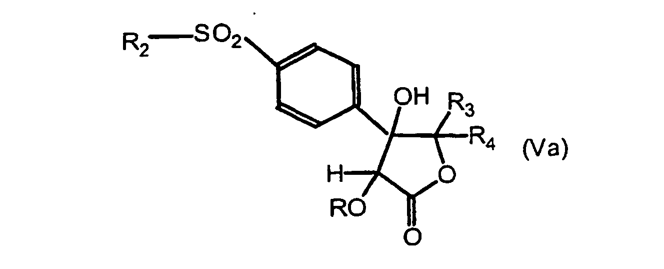 qui, après élimination d'une molécule d'eau forme un composé de formule générale la ; et
c) isolement du composé de formule générale la ainsi obtenu.

On préfère tout particulièrement les composés de formule générale la dans lesquels
- R représente le groupe cyclopropylméthyle et
- R₂, R₃ et R₄ représentent le groupe méthyle.

Dans un second mode de réalisation de l'invention, le groupe R₁ est un noyau phényle substitué.

Le composé de formule générale I devient alors un composé de formule générale Ib: 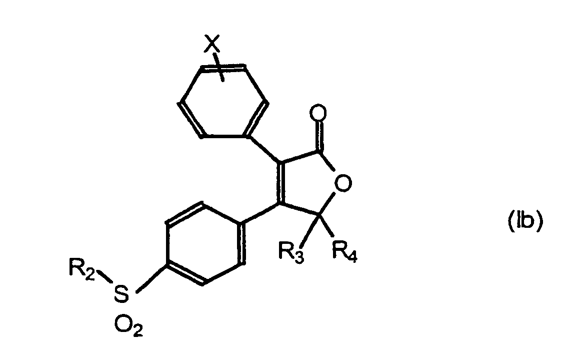 dans laquelle R₂ est tel que défini précédemment et X est choisi parmi :
- hydrogène ;
- halogène;
- (C₁-C₃) alkoxy ;
- CN;
- (C₁-C₃) fluoroalkyle ;
- (C₁-C₃)alkyle ;
- -COOH ;

Le procédé de l'invention comprend alors les étapes suivantes :
a) réaction d'un composé de formule générale II 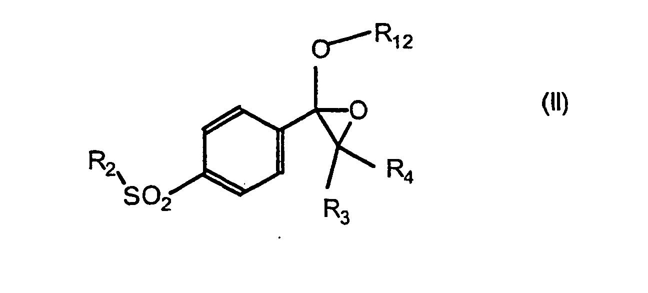 dans laquelle R₂, R₃ et R₄ sont tels que définis précédemment et R₁₂ représente un groupe alkyle en C₁-C₆,
   avec un acide de formule générale III b) 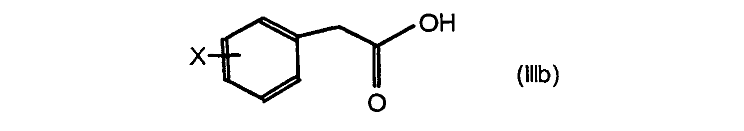 dans laquelle X est tel que défini précédemment, en milieu anhydre, pour former un composé de formule générale IV b) 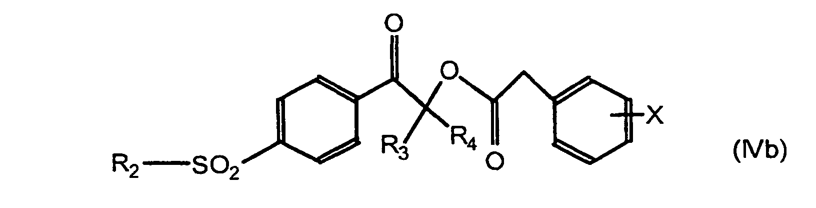 R₂, R₃ et R₄ étant tels que définis ci-dessus ;
b) réaction du composé de formule IVb avec une base forte dans un solvant aprotique pour obtenir un composé cyclique intermédiaire de formule Vb : 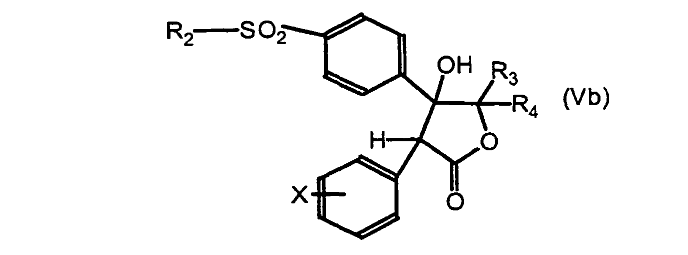 qui, après élimination d'une molécule d'eau forme un composé de formule générale Ib ;
c) isolement du composé de formule générale Ib ainsi obtenu.

Le composé intermédiaire de formule générale VI est nouveau et constitue un autre objet de l'invention.

On préfère en particulier les composés de formule générale VI dans lesquels R₂, R₃ et R₄ représentent le groupe méthyle et R₁₂ est tel que défini ci-dessus.

Un composé particulièrement préféré de ce type est celui dans lequel R₁₂ représente méthyle.

Le procédé de l'invention est illustré grâce à l'exemple suivant :

### Exemple : préparation de la 3-(cyclopropylméthoxy)-[4-(4-méthylsulfonyl) phényl)]-5,5-diméthyl-5-H-furan-2-one.

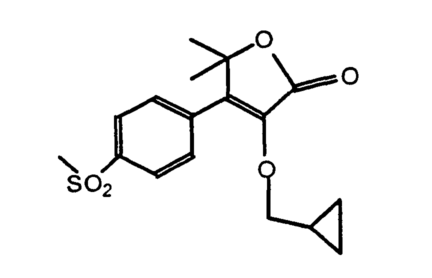

### 1) Préparation du 1-méthoxy-2-méthyl-1-(4'-méthylthiophényl)prop-1-ène :

A une solution de 2-méthyl-1-(4'-méthylthiophényl)propan-1-one (ou 4-thiométhyl-isobutyrophénone (composé 2 ; 10,11 g, 52 mmol, 1 équiv.) obtenu comme décrit à l'exemple 1 de WO 97/45420) par réaction de thioanisole en présence d'un acide de Lewis avec le chlorure d'isobutyryle dans un mélange méthanol (40 ml) / orthoformiate de méthyle (40 ml) est ajoutée l'acide p-toluène sulfonique (1,2 g, 6,3 mmol, 0,12 équiv.). Cette solution est chauffée 1,5 h à reflux puis le méthanol est distillé. La masse réactionnelle est ensuite chauffée 41 h à 115°C. Après retour à température ambiante, le mélange réactionnel est dilué avec du dichlorométhane (50 ml), lavé avec une solution aqueuse saturée de carbonate de potassium (50 ml) puis avec de la saumure (50 ml) et séchée sur sulfate de sodium. L'évaporation des solvants fournit un mélange de 1-méthoxy-2-méthyl-1-(4'-méthylthiophényl)prop-1-ène escompté et du 1,1-diméthoxy-2-méthyl-1-(4'-méthylthiophényl)propane dans un rapport molaire de 83/17 (10,0 g).
- RMN-¹H (200 MHz, CDCl₃) ppm : 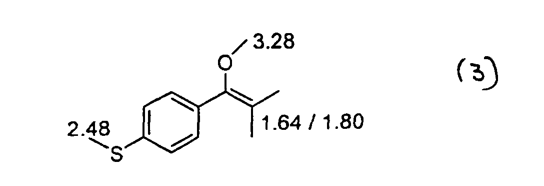
- GC/IR/MS:
   m/z : 208
   IR (cm⁻¹): aromatique C-H 3073; O-CH₃ 2872, 2840; C=C 1668, C-O-C 1143.

### 2) Préparation du 3,3-diméthyl-2-méthoxy-2-(4'-méthylsulfonylphenyl) oxirane :

A une suspension d'hydrogénocarbonate de sodium (38.14 g, 454,0 mmol) dans un mélange acétone (126 ml) / eau (167 ml) à 0°C est ajouté l'Oxone® (diméthyldioxirane) (78,9 g, 128,3 mmol, 6,3 équiv.) en 5 portions à intervalle de 3 minutes. Une solution de 1-méthoxy-2-méthyl-1-(4'-méthylthiophényl)prop-1-ène brut (4,24 g, 20,3 mmol) dans du dichlorométhane (10 ml) est additionnée au mélange réactionnel. Le bain de glace est retiré et le mélange est agité 3,5 h à température ambiante. Le milieu réactionnel est ensuite filtré et le filtrat est extrait au dichlorométhane (5x60 ml). Les phases organiques rassemblées sont séchées sur sulfate de sodium et concentrées pour fournir le 3,3-diméthyl-2-méthoxy-2-(4'-méthylsulfonylphenyl)oxirane escompté (composé 4 ; 3,80 g).
- RMN-¹H (200 MHz, CDCl₃) ppm: 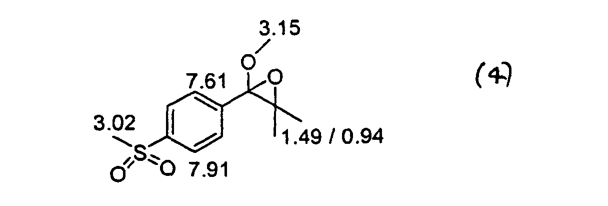
- GC/IR/MS:
   m/z : 241 (M-15); 183
   IR (cm⁻¹): O-CH₃ 2845; SO₂ 1348, 1164

### 3) Préparation du 2-(cyclopropylméthoxy)acétate de [2-méthyl-1-(4'-méthylsulfonylphényl)-1-oxo-prop-2-yle]:

Une solution de 3,3-diméthyl-2-méthoxy-2-(4'-méthylsulfonylphenyl)oxirane brut (3,6 g, 14,1 mmol) et d'acide 2-(cyclopropyl-méthoxy)acétique (2,17 g, 16,7 mmol, 1,2 équiv.) dans du *tert*-butylméthyléther anhydre (10 ml) est agitée à température ambiante pendant 2 jours. Le mélange réactionnel est ensuite concentré pour fournir un solide jaune (5,24 g) contenant 65% p/p de 2-(cyclopropylméthoxy)acétate de [2-méthyl-1-(4'-méthylsulfonylphényl)-1-oxo-prop-2-yle. Le rendement enchaîné à partir de la 2-méthyl-1-(4'-méthylthiophényl)propan-1-one est de 46% pur/pur.
- GC/IR/MS:
   m/z : 238; 183
   IR (cm⁻¹): C=O 1752, 1711; SO₂ 1349, 1166; C-O-C 1124

### 4) Préparation du 3-(cyclopropylméthoxy)-5.5-diméthyl-4-(4'-méthylsulfonylphényt)-5H-furan-2-one (composé du titre)

Une solution de trifluoroacétate d'isopropyle (1,58 g, 10,1 mmol, 1,2 équiv.) et de 1,8-diazabicyclo-[5.4.0]-undec-7-ène (2,6 g, 13,5 mmol, 1,6 équiv.) dans de l'acétonitrile anhydre (20 ml) est agitée 15 minutes à température ambiante. Le 2-(cyclopropylméthoxy)acétate de [2-méthyl-1-(4'-méthylsulfonylphényl)-1-oxo-prop-2-yle] (3,00 g, 8,4 mmol) est ensuite additionné et le mélange réactionnel est chauffé au reflux pendant 18 heures. Après retour de la température à 40°C, l'acétonitrile est partiellement évaporé puis de l'eau (20 ml) est rajoutée au milieu réactionnel. Après retour à température ambiante et quelques heures de cristallisation, le mélange est filtré pour récupérer la 3-(cyclopropylméthoxy)-5,5-diméthyl-4-(4'-méthylsulfonylphényl)-5H-furan-2-one escomptée. Le rendement en produit isolé est de 85%.
- RMN-¹H (200 MHz, CD₃COCD₃) ppm:

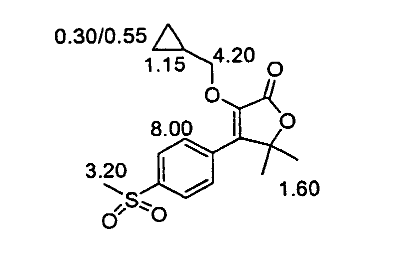

## Revendications

1. Procédé de préparation de composés de formule générale I : 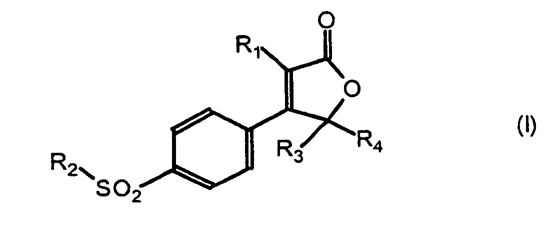 dans laquelle
• R₁ est choisi parmi les groupes
(a) OR₅ où R₅ représente un groupe choisi parmi
(1) un groupe C₁-C₆ alkyle linéaire ramifié ou cyclique ;
(2) un groupe phényle ou naphtyle mono-, di- ou tri-substitué
dans lesquels les substituants sont choisis parmi :
- hydrogène ;
- halogène ;
- (C₁-C₃) alkoxy ;
- CN;
- (C₁-C₃) fluoroalkyle ;
- (C₁-C₃)alkyle ;
- -COOH ;
et
(b) phényle mono-, di- ou tri-substitué dans lequel les substituants sont choisis parmi :
- hydrogène ;
- halogène ;
- (C₁-C₃) alkoxy ;
- CN ;
- (C₁-C₃) fluoroalkyle ;
- (C₁-C₃)alkyle ;
- -COOH ;
• R₂ représente un groupe (C₁-C₆)alkyle ;
• R₃ et R₄ représentent indépendamment l'un de l'autre un atome d'hydrogène ou un groupe CHR₆R₇
dans lequel R₆ et R₇ sont indépendamment l'un de l'autre choisis parmi :
- hydrogène ;
- (C₁-C₁₀)alkyle ;
- (C₁-C₁₀) alkoxy ;
- OH ;
- CN ;
- CH₂CN ;
- OCOR₈ ;
- (C₁-C₆)fluoroalkyle ;
- halogène ;
- CON(R₈)₂ ;
- phényle mono-, di- ou tri-substitué ;
- hétéroaryle mono-, di- ou tri-substitué ;
les substituants étant choisis parmi :
- hydrogène ;
- halogène ;
- (C₁-C₆)alkyle ;
- (C₁-C₁₀)alkoxy ;
- CN ;
- CF₃ ;
- N₃ ;
- C(R₉)(R₁₀)-OH ;
- C(R₉) (R₁₀)-O-(C₁-C₄)alkyle ;
- (C₁-C₆)fluoroalkyle ;
• R₈ est choisi parmi :
- hydrogène ;
- (C₁-C₆)alkyle ;
- phényle mono-, di- ou trisubstitué, les substituants étant choisis parmi hydrogène, halogène, (C₁-C₆)alkyle, (C₁-C₆)alkoxy, (C₁-C₆)alkylthio, CN ou CF₃; et
- benzyle mono-, di- ou trisubstitué, les substituants étant choisis parmi hydrogène, halogène, (C₁-C₆)alkyle, (C₁-C₆)alkoxy, (C₁-C₆)alkylthio, CN ou CF₃ ;
• ou deux groupes R₈ forment ensemble avec l'atome d'azote auxquels ils sont attachés un cycle ayant de 5 à 7 atomes, et comprenant éventuellement un hétéroatome choisi parmi O, S ou NR₉ ;
• R₉ et R₁₀ sont indépendamment l'un de l'autre choisis parmi :
- hydrogène ; et
- (C₁-C₁₀)alkyle ; ou
forment ensemble avec l'atome auquel ils sont attachés un cycle ayant de 3 à 7 atomes de carbone et le cas échéant un atome d'azote ;
**caractérisé en ce qu'**il comprend les étapes suivantes :
a) réaction d'un composé de formule générale II : 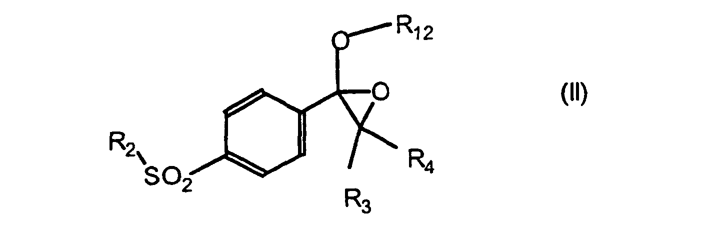 dans laquelle R₂, R₃ et R₄ sont tels que définis précédemment et
R₁₂ représente un groupe alkyle en C₁-C₆,
avec un acide de formule générale III : 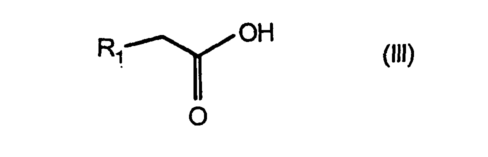 dans laquelle R₁ est tel que défini précédemment, en milieu anhydre, pour former un composé de formule IV : 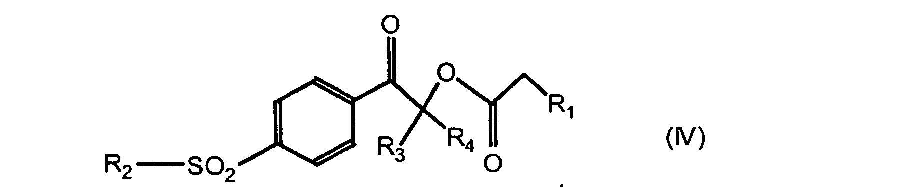 R₁,R₂, R₃ et R₄ étant tels que définis ci-dessus;
b) réaction du composé de formule IV avec une base forte dans un solvant aprotique pour obtenir un composé cyclique intermédiaire de formule V : 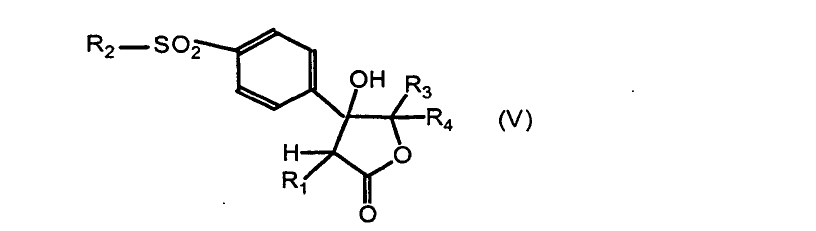 qui, après élimination d'une molécule d'eau forme un composé de formule générale 1 ;
c) isolement du composé de formule générale I ainsi obtenu.

2. Procédé selon la revendication 1, **caractérisé en ce que** l'on prépare le composé de formule générale II par réaction d'un composé de formule générale VI : 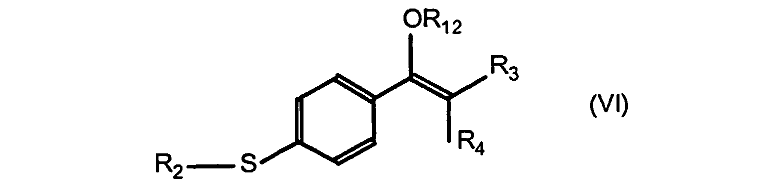 dans laquelle, R₂, R₃, R₄ et R₁₂ sont tels que définis à la revendication 1, avec un agent oxydant.

3. Procédé selon la revendication 2, **caractérisé en ce que** l'on prépare le composé de formule générale VI telle que définie à la revendication 2, par réaction d'un composé de formule générale VII : 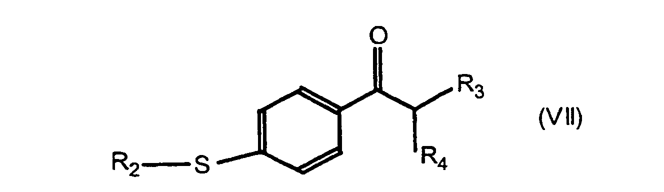 dans laquelle R₂, R₃ et R₄ sont tels que définis à la revendication 1, avec un alcool de formule générale VIII :
HO R₁₂ (VIII)
R₁₂ étant tel que défini ci-dessus, en présence d'une quantité catalytique d'acide et d'un agent déshydratant.

4. Procédé selon la revendication 3, **caractérisé en ce que** R₁₂ est méthyle et le composé de formule générale VIII est le méthanol.

5. Procédé de préparation d'un composé de formule générale I tel que défini à la revendication 1, **caractérisé en ce qu'**il comprend les étapes suivantes :
(1) réaction d'un composé de formule générale IX : 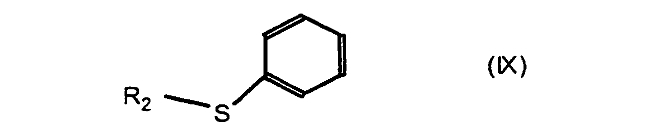 dans laquelle R₂ est tel que défini à la revendication 1, dans un solvant inerte en présence d'un acide de Lewis avec un composé de formule générale X : 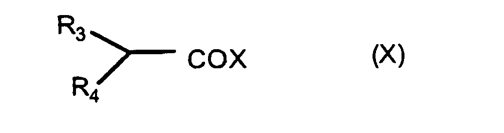 dans laquelle X est un groupe partant, de préférence un atome de chlore pour former un composé de formule générale VII : 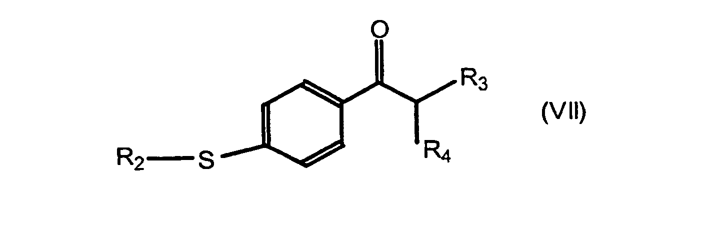 dans laquelle R₂, R₃ et R₄ sont tels que définis à la revendication 1,
(2) réaction du composé de formule générale VII avec un alcool de formule générale VIII :
R₁₂-OH (VIII)
dans laquelle R₁₂ représente un groupe (C₁-C₆)alkyle pour former un composé de formule générale VI : 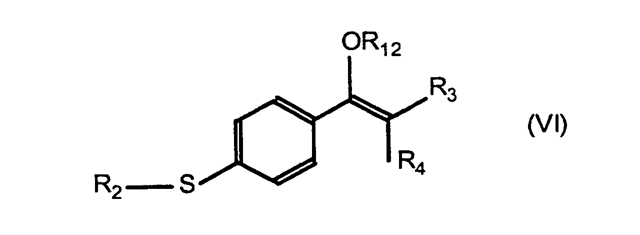 dans laquelle R₂, R₃, R₄ et R₁₂ sont tels que définis précédemment,
(3) réaction du composé de formule générale VI avec un agent oxydant pour obtenir un composé de formule générale II : 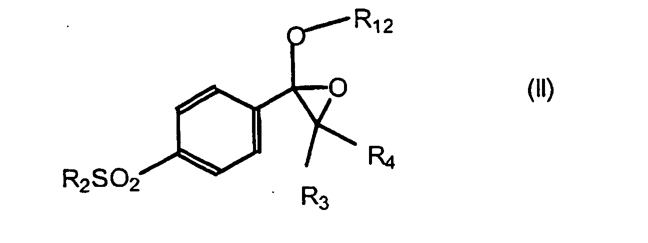 dans laquelle R₂, R₃, R₄ et R₁₂ sont tels que définis précédemment;
(4) réaction du composé de formule générale II telle que définie à l'étape (3)
avec un acide de formule générale III : 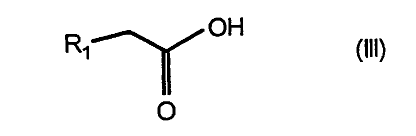 dans laquelle R₁ est tel que défini précédemment, en milieu anhydre, pour former un composé de formule IV : 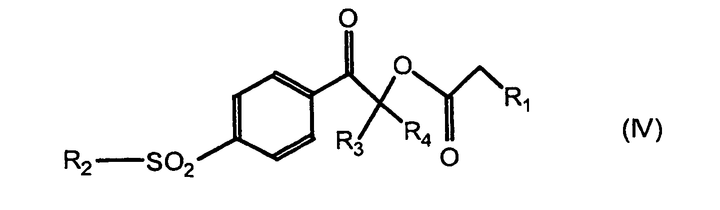 R₁,R₂, R₃ et R₄ étant tels que définis ci-dessus;
(5) réaction du composé de formule IV avec une base forte dans un solvant aprotique pour obtenir un composé cyclique intermédiaire de formule V : 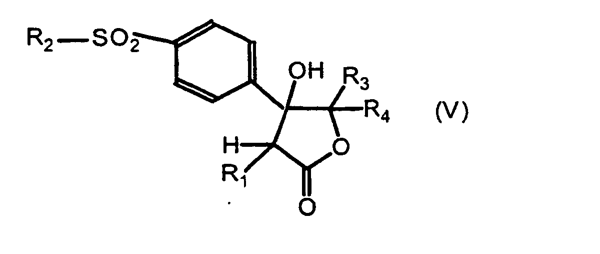 qui, après élimination d'une molécule d'eau forme un composé de formule générale I ;
(6) isolement du composé de formule générale I ainsi obtenu.

6. Procédé selon la revendication 1 de préparation d'un composé de formule générale la : 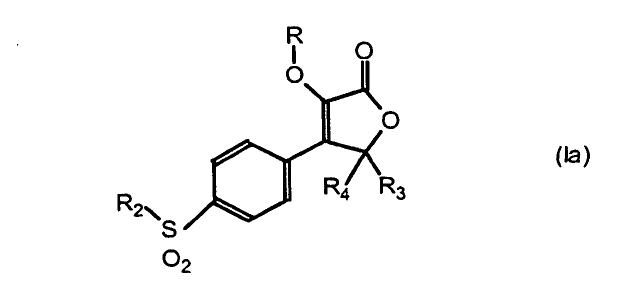 dans laquelle R représente un groupe R₅ tel que défini à la revendication 1 et R₂, R₃ et R₄ sont tels que définis à la revendication 1, **caractérisé en ce qu'**il comprend les étapes suivantes :
a) réaction d'un composé de formule générale II : 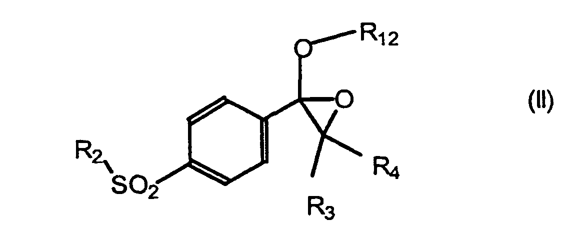 dans laquelle R₂, R₃ et R₄ sont tels que définis précédemment et R₁₂ représente un groupe alkyle en C₁-C₆,
avec un acide de formule générale IIIa : 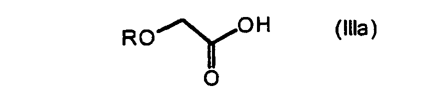 dans laquelle R est tel que défini ci-dessus, en milieu anhydre pour former un composé de formule générale IVa : 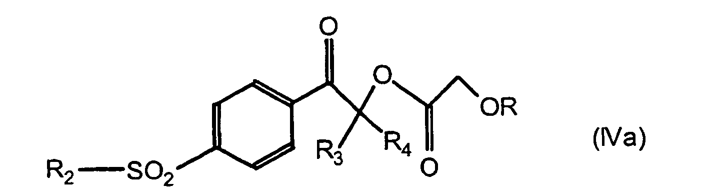 dans laquelle R, R₂, R₃ et R₄ sont tels que définis précédemment.
b) réaction du composé de formule IVa avec une base forte dans un solvant aprotique pour obtenir un composé cyclique intermédiaire de formule Va : 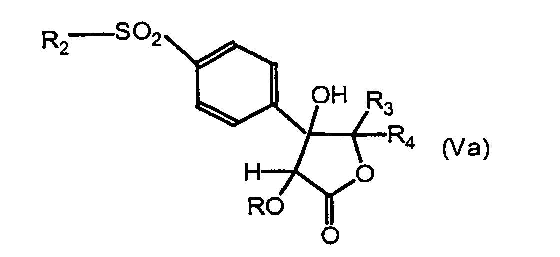 qui, après élimination d'une molécule d'eau forme un composé de formule générale Ia ;
c) isolement du composé de formule générale la ainsi obtenu.

7. Procédé selon la revendication 1 de préparation d'un composé de formule générale Ib : 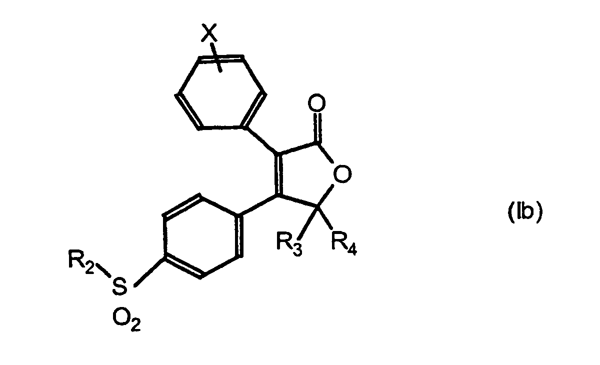 dans laquelle R₂ est tel que défini à la revendication 1 et X est choisi parmi :
- hydrogène ;
- halogène ;
- (C₁-C₃) alkoxy ;
- CN;
- (C₁-C₃) fluoroalkyle ;
- (C₁-C₃)alkyle ;
- -COOH ;
**caractérisé en ce qu'**il comprend les étapes suivantes :
a) réaction d'un composé de formule générale II 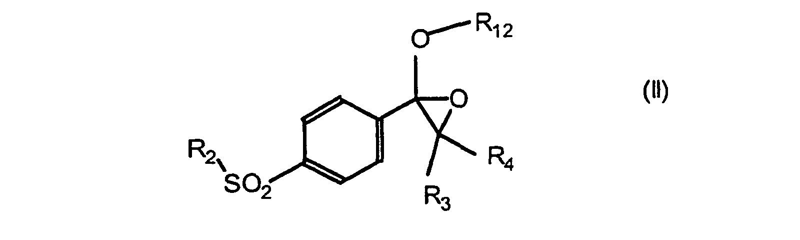 dans laquelle R₂, R₃ et R₄ sont tels que définis précédemment et R₁₂ représente un groupe alkyle en C₁-C₆,
avec un acide de formule générale III b) 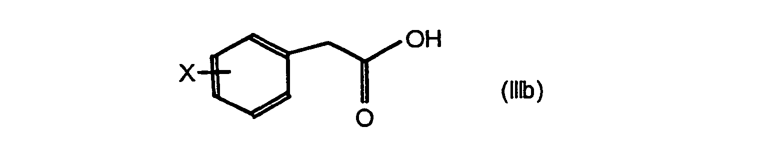 dans laquelle X est tel que défini précédemment, en milieu anhydre, pour former un composé de formule générale IV b) 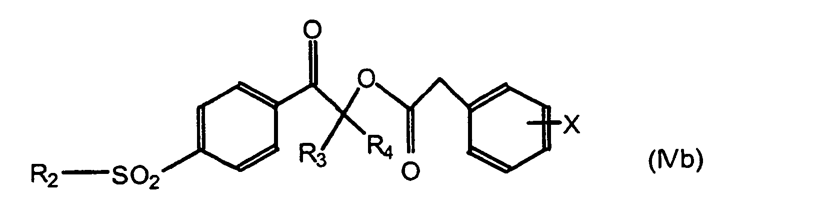 R₂, R₃ et R₄ étant tels que définis ci-dessus ;
b) réaction du composé de formule IVb avec une base forte dans un solvant aprotique pour obtenir un composé cyclique intermédiaire de formule Vb : 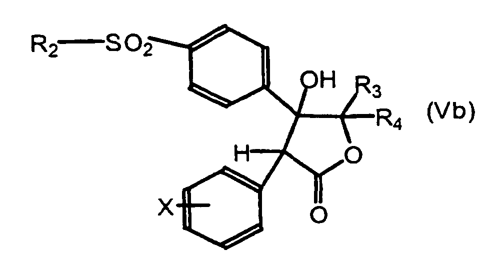 qui, après élimination d'une molécule d'eau forme un composé de formule générale Ib ;
c) isolement du composé de formule générale Ib ainsi obtenu.

8. Procédé de préparation du composé de formule (1) : 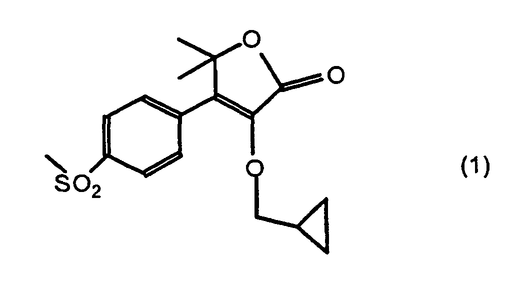 comportant les étapes suivantes :
- réaction du thioanisole en présence d'un acide de Lewis avec le chlorure d'isobutyryle pour obtenir le composé de formule (2) 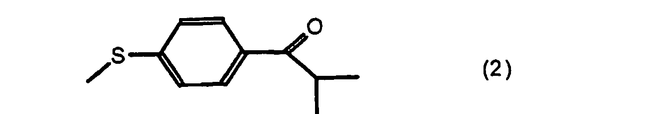 réaction du composé de formule (2) avec le méthanol en présence d'acide para-toluène sulfonique et d'orthoformiate de méthyle pour former le composé de formule (3) : 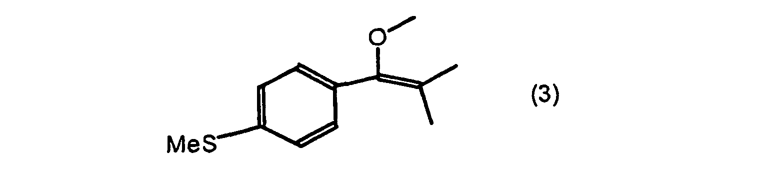
- réaction du composé (3) avec du diméthyldioxirane pour former le composé de formule (4) : 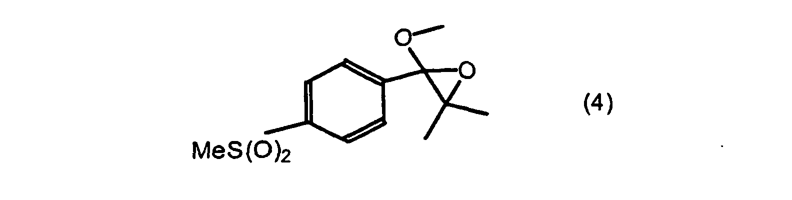 réaction du composé (4) avec de l'acide cyclopropylméthyloxy acétique dans un solvant anhydre pour obtenir le composé (5) : 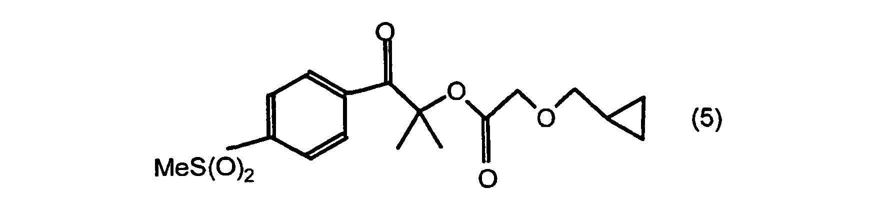
- réaction du composé (5) dans un solvant aprotique avec une base forte pour obtenir un composé cyclique intermédiaire (6) : 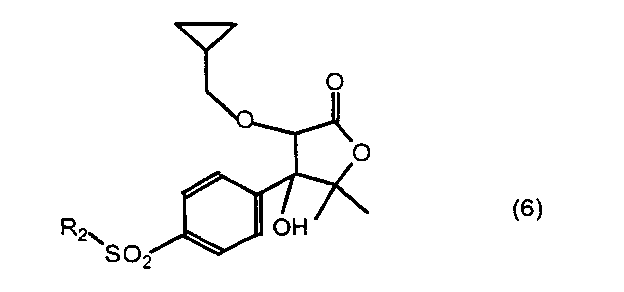 qui, après déhydratation en présence d'un agent déshydratant forme le composé (1).

9. Procédé selon la revendication 2 ou 5, **caractérisé en ce que** l'agent oxydant est choisi parmi les peracides organiques, et les dioxiranes.

10. Procédé selon la revendication 2 ou 5, **caractérisé en ce que** la température de la réaction d'oxydation est comprise entre -40° et 30°C.

11. Procédé selon la revendication 2 ou 5, **caractérisé en ce que** l'agent oxydant est utilisé en excès par rapport au composé de formule générale II.

12. Procédé selon la revendication 3, **caractérisé en ce que** l'acide est choisi parmi les acides sulfoniques et les acides minéraux.

13. Procédé selon la revendication 3, **caractérisé en ce que** à titre d'agent déshydratant, on utilise un orthoformiate d'alkyle en C₁-C_{6.}

14. Procédé selon la revendication 3 ou 5, **caractérisé en ce que** la réaction est réalisée dans un excès d'alcool de formule générale VIII, servant de solvant réactif.

15. Composé de formule générale VI : 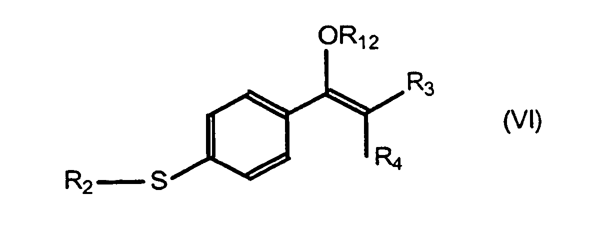 dans lequel
• R₂ représente un groupe (C₁-C₆)alkyle ;
• R₃ et R₄ représentent indépendamment l'un de l'autre un atome d'hydrogène ou un groupe CHR₆R₇ dans lequel R₆ et R₇ sont indépendamment l'un de l'autre choisis parmi :
- hydrogène ;
- (C₁-C₁₀)alkyle ;
- (C₁-C₁₀) alkoxy ;
- OH ;
- CN ;
- CH₂CN ;
- OCOR₈ ;
- (C₁-C₆)fluoroalkyle ;
- halogène ;
- CON(R₈)₂ ;
- phényle mono-, di- ou trisubstitué ;
- hétéroaryle mono-, di- ou trisubstitué ;
les substituants étant choisis parmi :
- hydrogène ;
- halogène ;
- (C₁-C₆)alkyle ;
- (C₁-C₁₀)alkoxy ;
- CN;
- CF₃ ;
- N₃ ;
- C(R₉)(R₁₀)-OH ;
- C(R₉) (R₁₀)-O-(C₁-C₄)alkyle ;
- (C₁-C₆)fluoroalkyle ;
• R₈ est choisi parmi :
- hydrogène ;
- (C₁-C₆)alkyle ;
- phényle mono-, di- ou trisubstitué, les substituants étant choisis parmi hydrogène, halogène, (C₁-C₆)alkyle, (C₁-C₆)alkoxy, (Ci-C₆)alkylthio, CN ou CF₃; et
- benzyle mono-, di- ou trisubstitué, les substituants étant choisis parmi hydrogène, halogène, (C₁-C₆)alkyle, (C₁-C₆)alkoxy, (C₁-C₆)alkylthio, CN ou CF₃ ;
• ou deux groupes R₈ forment ensemble avec l'atome d'azote auxquels ils sont attachés un cycle ayant de 5 à 7 atomes, et comprenant éventuellement un hétéroatome choisi parmi O, S ou NR₉ ;
• R₉ et R₁₀ sont indépendamment l'un de l'autre choisis parmi :
- hydrogène ; et
- (C₁-C₁₀)alkyle ; ou
forment ensemble avec l'atome auquel ils sont attachés un cycle ayant de 3 à 7 atomes de carbone et le cas échéant un atome d'azote ;
et R₁₂ représente un groupe alkyle en C₁-C₆.

16. Composé de formule 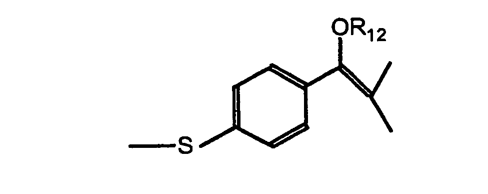 dans lequel R₁₂ est tel que défini à la revendication 15, notamment méthyle.

## Patentansprüche

1. Verfahren zur Herstellung von Verbindungen der allgemeinen Formel I: 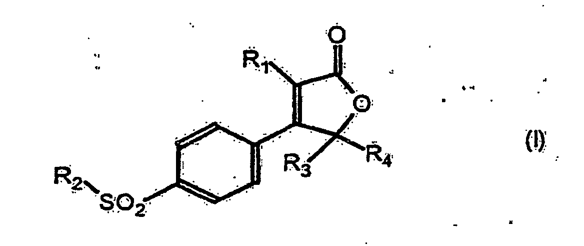 wobei
• R₁ ausgewählt ist aus den Resten
(a) OR₅, wobei R₅ einen Rest darstellt, der ausgewählt ist aus
(1) einem linearen, verzweigten oder cyclischen C₁-C₆-Alkylrest;
(2) einem mono-, di- oder trisubstituierten Phenyl- oder Naphthylrest, wobei die Substituenten ausgewählt sind aus:
- Wasserstoff;
- Halogen;
- (C₁-C₃)-Alkoxy;
- CN;
- (C₁-C₃)-Fluoralkyl;
- (C₁-C₃)-Alkyl;
- -COOH;
und
(b) mono-, di- oder trisubstituiertem Phenyl, wobei die Substituenten ausgewählt sind aus:
- Wasserstoff;
- Halogen;
- (C₁-C₃)-Alkoxy;
- CN;
- (C₁-C₃)-Fluoralkyl;
- (C₁-C₃)-Alkyl;
- -COOH;
• R₂ einen (C₁-C₆)-Alkylrest bedeutet;
• R₃ und R₄ unabhängig voneinander ein Wasserstoffatom oder einen Rest CHR₆R₇ bedeuten, wobei R₆ und R₇ unabhängig voneinander ausgewählt sind aus:
- Wasserstoff;
- (C₁-C₁₀)-Alkyl;
- (C₁-C₁₀)-Alkoxy;
- OH;
- CN;
- CH₂CN;
- OCOR₈;
- (C₁-C₆)-Fluoralkyl;
- Halogen;
- CON(R₈)₂;
- mono-, di- oder trisubstituiertem Phenyl;
- mono-, di- oder trisubstituiertem Heteroaryl;
wobei die Substituenten ausgewählt sind aus:
- Wasserstoff;
- Halogen;
- (C₁-C₆)-Alkyl;
- (C₁-C₁₀)-Alkoxy;
- CN;
- CF₃;
- N₃;
- C(R₉)(R₁₀)-OH;
- C(R₉)(R₁₀)-O-(C₁-C₄)-alkyl;
- (C₁-C₆)-Fluoralkyl;
• R₈ ausgewählt ist aus:
- Wasserstoff;
- (C₁-C₆)-Alkyl;
- mono-, di- oder trisubstituiertem Phenyl, wobei die Substituenten ausgewählt sind aus Wasserstoff, Halogen, (C₁-C₆)-Alkyl (C₁-C₆)-Alkoxy, (C₁-C₆)-Alkylthio, CN oder CF₃; und
- mono-, di- oder trisubstituiertem Benzyl, wobei die Substituenten ausgewählt sind aus Wasserstoff, Halogen, (C₁-C₆)-Alkyl, (C₁-C₆)-Alkoxy, (C₁-C₆)-Alkylthio, CN oder CF₃;
• oder zwei Reste R₈ zusammen mit dem Stickstoffatom, an das sie gebunden sind, einen Ring mit 5 bis 7 Atomen bilden und gegebenenfalls ein Heteroatom, ausgewählt aus O, S oder NR₉, umfassen;
• R₉ und R₁₀ unabhängig voneinander ausgewählt sind aus:
- Wasserstoff; und
- (C₁-C₁₀)-Alkyl; oder
zusammen mit dem Atom, an das sie gebunden sind, einen Ring mit 3 bis 7 Kohlenstoffatomen und gegebenenfalls einem Stickstoffatom bilden;
**dadurch gekennzeichnet, daß** es die folgenden Schritte umfaßt:
a) Umsetzen einer Verbindung der allgemeinen Formel II: 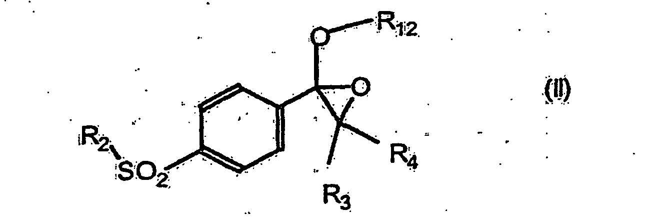 wobei R₂, R₃ und R₄ wie vorstehend definiert sind und R₁₂ einen C₁-C₆-Alkykest bedeutet, mit einer Säure der allgemeinen Formel III: 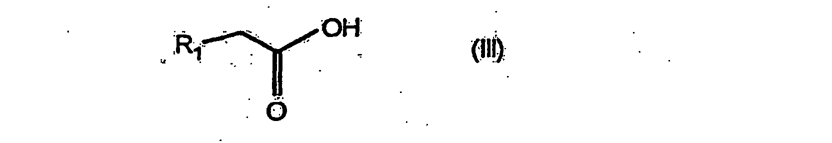 wobei R₁ wie vorstehend definiert ist, in wasserfreiem Medium unter Bildung einer Verbindung der Formel IV: 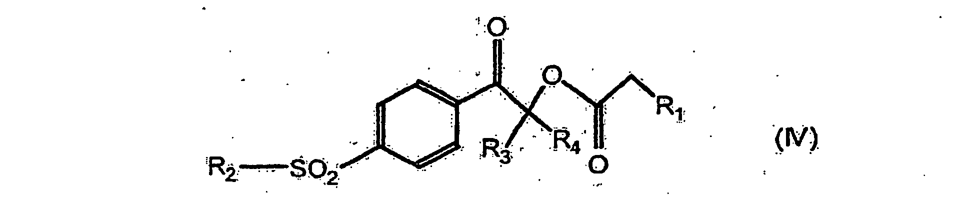 wobei R₁, R₂, R₃ und R₄ wie vorstehend definiert sind;
b) Umsetzen der Verbindung der Formel IV mit einer starken Base in einem aprotischen Lösungsmittel, um eine cyclische Zwischenverbindung der Formel V zu erhalten: 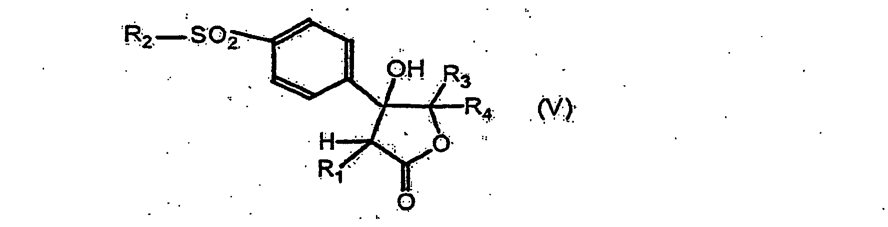 welche nach Abspaltung eines Wassermoleküls eine Verbindung der allgemeinen Formel I bildet;
c) Isolieren der so erhaltenen Verbindung der allgemeinen Formel I.

2. Verfahren nach Anspruch 1, **dadurch gekennzeichnet, daß** die Verbindung der allgemeinen Formel II durch Umsetzen einer Verbindung der allgemeinen Formel VI: 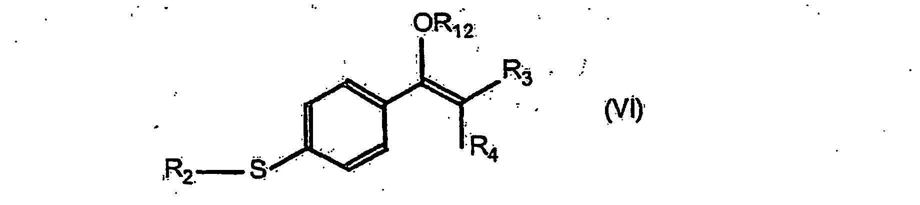 wobei R₂, R₃, R₄ und R₁₂ wie in Anspruch 1 definiert sind, mit einem Oxidationsmittel hergestellt wird.

3. Verfahren nach Anspruch 2, **dadurch gekennzeichnet, daß** die Verbindung der allgemeinen Formel VI, wie in Anspruch 2 definiert, durch Umsetzen einer Verbindung der allgemeinen Formel VII: 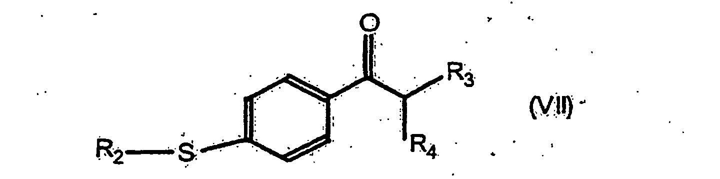 wobei R₂, R₃ und R₄ wie in Anspruch 1 definiert sind, mit einem Alkohol der allgemeinen Formel VIII:
HO R₁₂ (VIII)
wobei R₁₂ wie vorstehend definiert ist, in Gegenwart einer katalytischen Menge einer Säure und eines Dehydratisierungsmittels hergestellt wird.

4. Verfahren nach Anspruch 3, **dadurch gekennzeichnet, daß** R₁₂ Methyl ist und die Verbindung der allgemeinen Formel VIII Methanol ist.

5. Verfahren zur Herstellung einer Verbindung der allgemeinen Formel I, wie in Anspruch 1 definiert, **dadurch gekennzeichnet, daß** es die folgenden Schritte umfaßt:
(1) Umsetzen einer Verbindung der allgemeinen Formel IX: 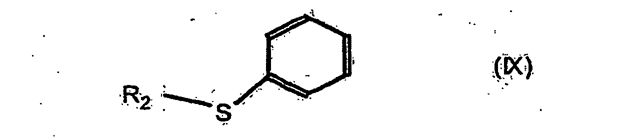 wobei R₂ wie in Anspruch 1 definiert ist, in einem inerten Lösungsmittel in Gegenwart einer Lewissäure mit einer Verbindung der allgemeinen Formel X: 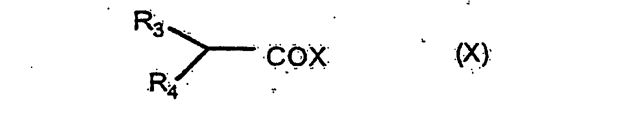 wobei X eine Abgangsgruppe, vorzugsweise ein Chloratom, ist, unter Bildung einer Verbindung der allgemeinen Formel VII: 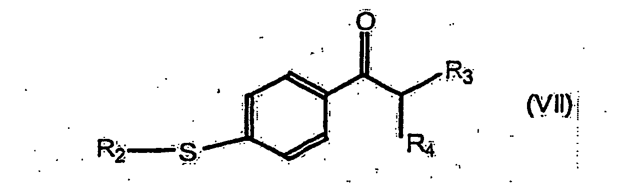 wobei R₂, R₃ und R₄ wie in Anspruch 1 definiert sind,
(2) Umsetzen der Verbindung der allgemeinen Formel VII mit einem Alkohol der allgemeinen Formel VIII:
R₁₂-OH (VIII)
wobei R₁₂ einen (C₁-C₆)-Alkylrest bedeutet, unter Bildung einer Verbindung der allgemeinen Formel VI: 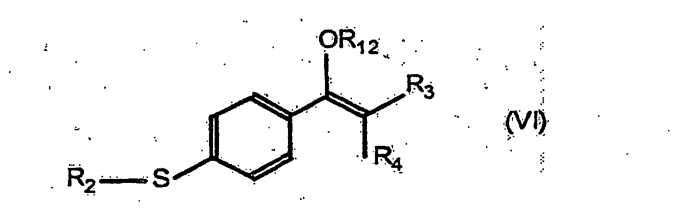 wobei R₂, R₃, R₄ und R₁₂ wie vorstehend definiert sind,
(3) Umsetzen der Verbindung der allgemeinen Formel VI mit einem Oxidationsmittel, um eine Verbindung der allgemeinen Formel II zu erhalten: 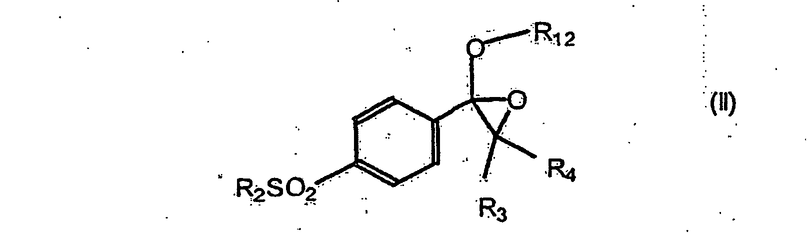 wobei R₂, R₃, R₄ und R₁₂ wie vorstehend definiert sind;
(4) Umsetzen der Verbindung der allgemeinen Formel II, wie in Schritt (3) definiert, mit einer Säure der allgemeinen Formel III: 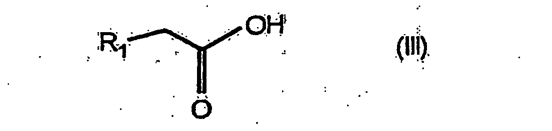 wobei R₁ wie vorstehend definiert ist, in wasserfreiem Medium unter Bildung einer Verbindung der Formel IV: 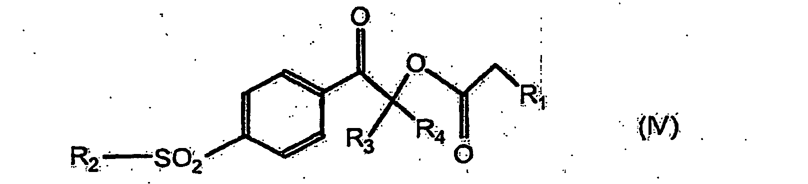 wobei R₁, R₂, R₃ und R₄ wie vorstehend definiert sind,
(5) Umsetzen der Verbindung der Formel IV mit einer starken Base in einem aprotischen Lösungsmittel, um eine cyclische Zwischenverbindung der Formel V zu erhalten: 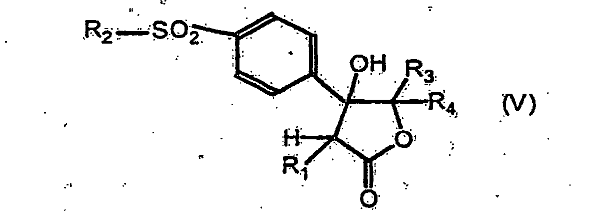 welche nach Abspaltung eines Wassermoleküls eine Verbindung der allgemeinen Formel I bildet;
(6) Isolieren der so erhaltenen Verbindung der allgemeinen Formel I.

6. Verfahren nach Anspruch 1 zur Herstellung einer Verbindung der allgemeinen Formel la: 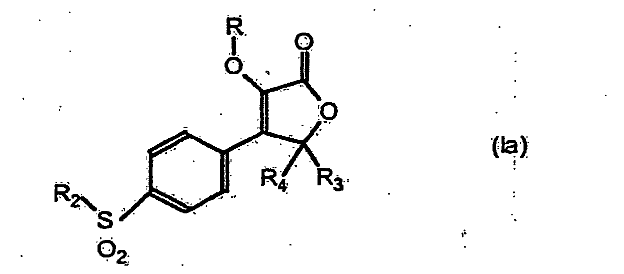 wobei R einen Rest R₅, wie in Anspruch 1 definiert, bedeutet und R₂, R₃ und R₄ wie in Anspruch 1 definiert sind, **dadurch gekennzeichnet, daß** es die folgenden Schritte umfaßt:
a) Umsetzen einer Verbindung der allgemeinen Formel II: 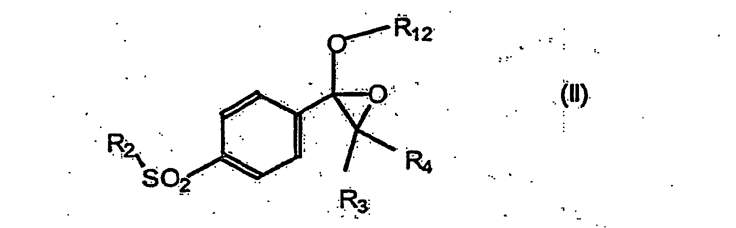 wobei R₂, R₃ und R₄ wie vorstehend definiert sind und R₁₂ einen C₁-C₆-Alkylrest bedeutet; mit einer Säure der allgemeinen Formel IIIa: 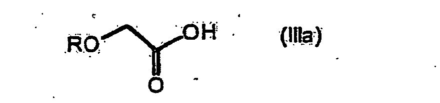 wobei R wie vorstehend definiert ist, in wasserfreiem Medium unter Bildung einer Verbindung der allgemeinen Formel IVa: 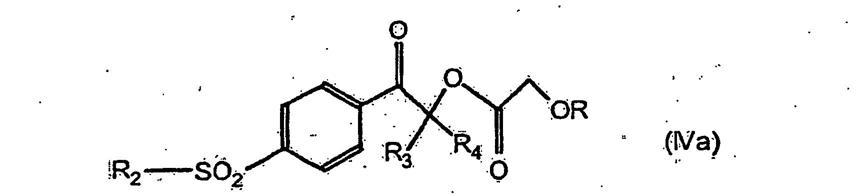 wobei R, R₂, R₃ und R₄ wie vorstehend definiert sind;
b) Umsetzen der Verbindung der Formel IVa mit einer starken Base in einem aprotischen Lösungsmittel, um eine cyclische Zwischenverbindung der Formel Va zu erhalten: 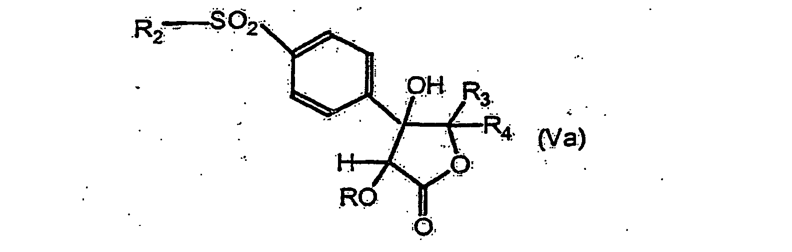 welche nach Abspaltung eines Wassermoleküls eine Verbindung der allgemeinen Formel Ia bildet;
c) Isolieren der so erhaltenen Verbindung der allgemeinen Formel Ia.

7. Verfahren nach Anspruch 1 zur Herstellung einer Verbindung der allgemeinen Formel Ib: 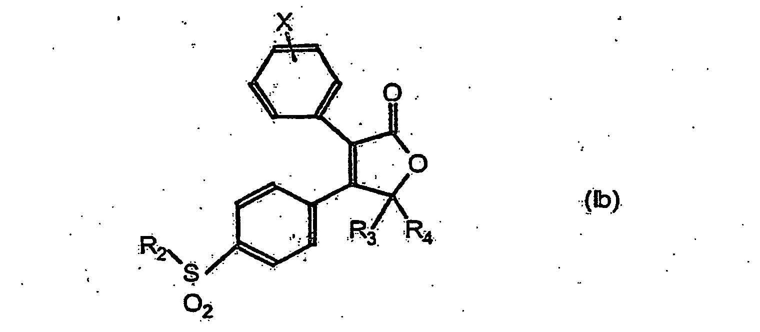 wobei R₂ wie in Anspruch 1 definiert ist und X ausgewählt ist aus:
- Wasserstoff;
- Halogen;
- (C₁-C₃)-Alkoxy;
- CN;
- (C₁-C₃)-Fluoralkyl;
- (C₁-C₃)-Alkyl;
- -COOH;
**dadurch gekennzeichnet, daß** es die folgenden Schritte umfaßt:
a) Umsetzen einer Verbindung der allgemeinen Formel II 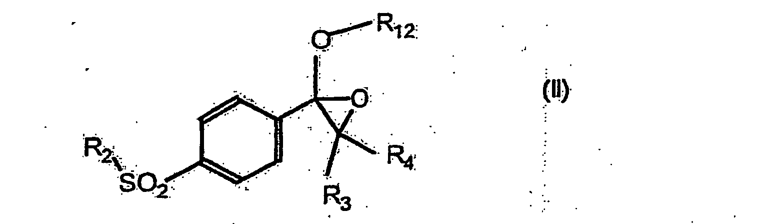 wobei R₂, R₃ und R₄ wie vorstehend definiert sind und R₁₂ einen C₁-C₆-Alkylrest bedeutet, mit einer Säure der allgemeinen Formel IIIb: 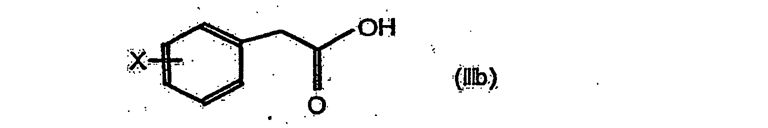 wobei X wie vorstehend definiert ist, in wasserfreiem Medium unter Bildung einer Verbindung der allgemeinen Formel IVb: 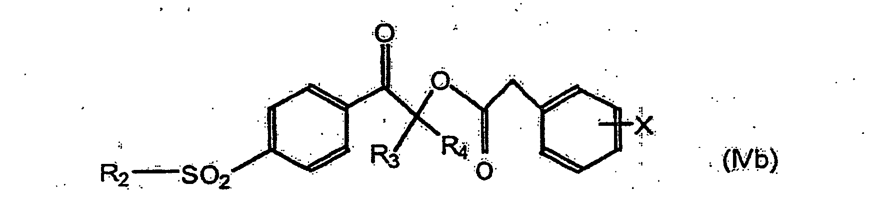 wobei R₂, R₃ und R₄ wie vorstehend definiert sind;
b) Umsetzen der Verbindung der Formel IVb mit einer starken Base in einem aprotischen Lösungsmittel, um eine cyclische Zwischenverbindung der Formel Vb zu erhalten: 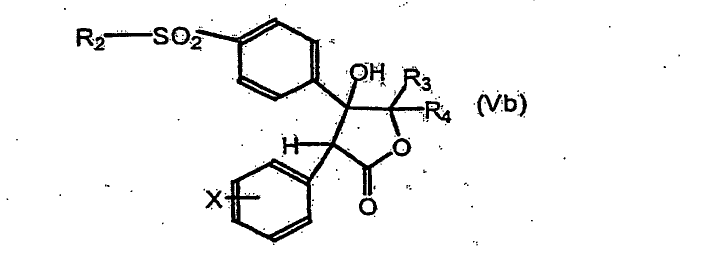 welche nach Abspaltung eines Wassermoleküls eine Verbindung der allgemeinen Formel Ib bildet;
c) Isolieren der so erhaltenen Verbindung der allgemeinen Formel Ib.

8. Verfahren zur Herstellung der Verbindung der Formel (1): 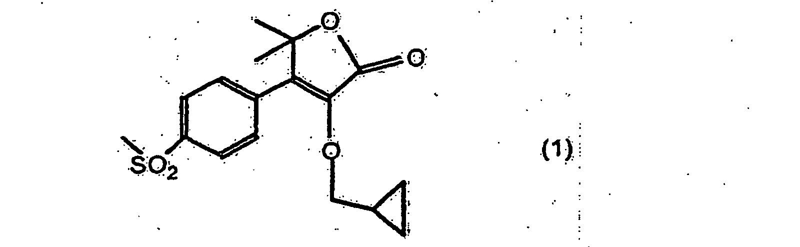 umfassend die folgenden Schritte:
- Umsetzen von Thioanisol in Gegenwart einer Lewissäure mit Isobutyrylchlorid, um die Verbindung der Formel (2) zu erhalten 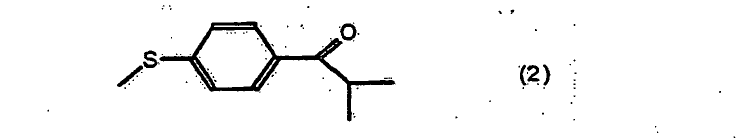
- Umsetzen der Verbindung der Formel (2) mit Methanol in Gegenwart von para-Toluolsulfonsäure und Methylorthoformiat unter Bildung der Verbindung der Formel (3): 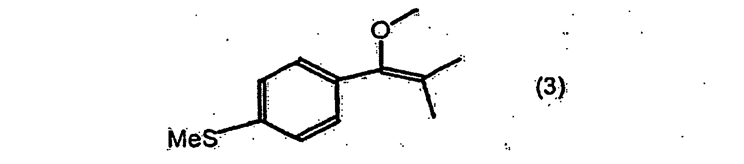
- Umsetzen der Verbindung (3) mit Dimethyldioxiran unter Bildung der Verbindung der Formel (4): 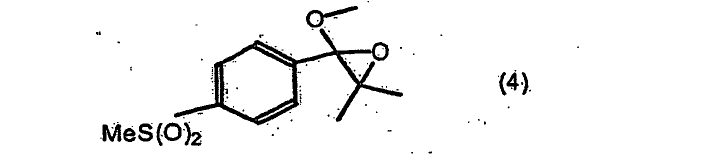
- Umsetzen der Verbindung (4) mit. Cyclopropylmethyloxyessigsäure in einem wasserfreien Lösungsmittel, um die Verbindung (5) zu erhalten: 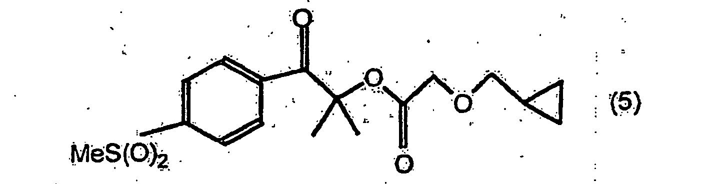
- Umsetzen der Verbindung (5) in einem aprotischen Lösungsmittel mit einer starken Base, um eine cyclische Zwischenverbindung (6) zu erhalten: 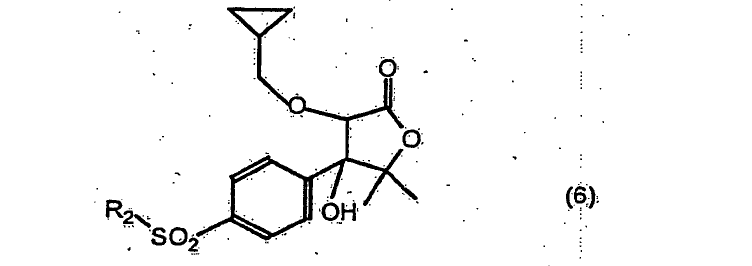 welche nach der Dehydratisierung in Gegenwart eines Dehydratisierungsmittels die Verbindung (1) bildet.

9. Verfahren nach Anspruch 2 oder 5, **dadurch gekennzeichnet, daß** das Oxidationsmittel aus organischen Persäuren und Dioxiranen ausgewählt ist.

10. Verfahren nach Anspruch 2 oder 5, **dadurch gekennzeichnet, daß** die Temperatur der Oxidationsreaktion zwischen -40°C und 30°C liegt.

11. Verfahren nach Anspruch 2 oder 5, **dadurch gekennzeichnet, daß** das Oxidationsmittel im Überschuß, bezogen auf die Verbindung der allgemeinen Formel II, verwendet wird.

12. Verfahren nach Anspruch 3, **dadurch gekennzeichnet, daß** die Säure aus Sulfonsäuren und Mineralsäuren ausgewählt ist.

13. Verfahren nach Anspruch 3, **dadurch gekennzeichnet, daß** als Dehydratisierungsmittel ein C₁-C₆-Alkylorthoformiat verwendet wird.

14. Verfahren nach Anspruch 3 oder 5, **dadurch gekennzeichnet, daß** die Reaktion in einem Überschuß an Alkohol der allgemeinen Formel VIII, der als reaktives Lösungsmittel dient, durchgeführt wird.

15. Verbindung der allgemeinen Formel VI: 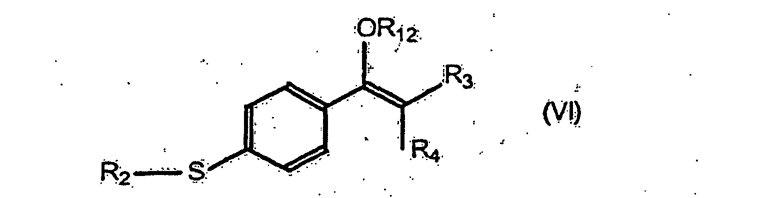 wobei
• R₂ einen (C₁-C₆)-Alkylrest bedeutet;
• R₃ und R₄ unabhängig voneinander ein Wasserstoffatom oder einen Rest CHR₆R₇ bedeuten, wobei R₆ und R₇ unabhängig voneinander ausgewählt sind aus:
- Wasserstoff;
- (C₁-C₁₀)-Alkyl;
- (C₁-C₁₀)-Alkoxy;
- OH;
- CN;
- CH₂CN;
- OCOR₈;
- (C₁-C₆)-Fluoralkyl;
- Halogen;
- CON(R₈)₂;
- mono-, di- oder trisubstituiertem Phenyl;
- mono-, di- oder trisubstituiertem Heteroaryl;
wobei die Substituenten asugewählt sind aus:
- Wasserstoff;
- Halogen;
- (C₁-C₆)-Alkyl;
- (C₁-C₁₀)-Alkoxy;
- CN;
- CF₃;
- N₃;
- C(R₉)(R₁₀)-OH;
- C(R₉)(R₁₀)-O-(C₁-C₄)-Alkyl;
- (C₁-C₆)-Fluoralkyl;
• R₈ ausgewählt ist aus:
- Wasserstoff;
- (C₁-C₆)-Alkyl;
- mono-, di- oder trisubstituiertem Phenyl, wobei die Substituenten ausgewählt sind aus Wasserstoff, Halogen, (C₁-C₆)-Alkyl, (C₁-C₆)-Alkoxy, (C₁-C₆)-Alkylthio, CN oder CF₃; und
- mono-, di- oder trisubstituiertem Benzyl, wobei die Substituenten ausgewählt sind aus Wasserstoff, Halogen, (C₁-C₆)-Alkyl, (C₁-C₆)-Alkoxy, (C₁-C₆)-Alkylthio, CN oder CF₃;
• oder zwei Reste R₈ zusammen mit dem Stickstoffatom, an das sie gebunden sind, einen Ring mit 5 bis 7 Atomen bilden und gegebenenfalls ein Heteroatom, ausgewählt aus O, S oder NR₉, umfassen;
• R₉ und R₁₀ unabhängig voneinander ausgewählt sind aus:
- Wasserstoff; und
- (C₁-C₁₀)-Alkyl; oder
zusammen mit dem Atom, an das sie gebunden sind, einen Ring mit 3 bis 7 Kohlenstoffatomen und gegebenenfalls einem Stickstoffatom bilden; und R₁₂ einen C₁-C₆-Alkylrest bedeutet.

16. Verbindung der Formel 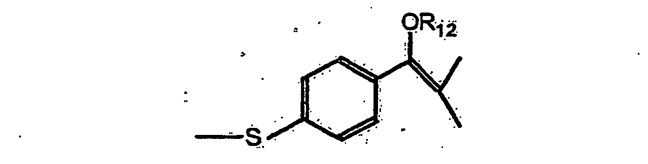 wobei R₁₂ wie in Anspruch 15 definiert, insbesondere Methyl, ist.

## Claims

1. Process for the preparation of compounds of general formula I: 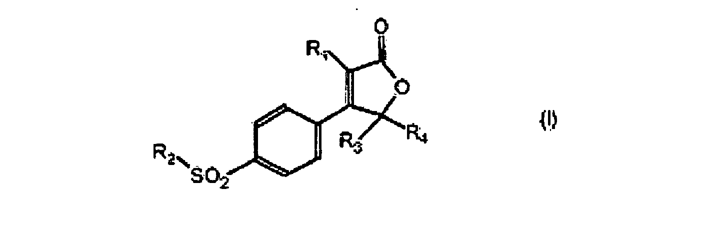 in which
• R₁ is chosen from the groups
(a) OR₅ where R₅ represents a group chosen from
(1) a linear, branched or cyclic C₁-C₆ alkyl group;
(2) a mono-, di- or tri-substituted phenyl or naphthyl group in which the substituents are chosen from:
- hydrogen;
- halogen;
- (C₁-C₃)alkoxy;
- CN;
- (C₁-C₃)fluoroalkyl;
- (C₁-C₃)alkyl;
- -COOH;
and
(b) mono-, di- or tri-substituted phenyl in which the substituents are chosen from:
- hydrogen;
- halogen;
- (C₁-C₃)alkoxy;
- CN;
- (C₁-C₃)fluoroalkyl;
- (C₁-C₃)alkyl;
- -COOH;
• R₂ represents a (C₁-C₆)alkyl group
• R₃ and R₄ independently of one another represent a hydrogen atom or a CHR₆R₇ group
in which R₆ and R₇ independently of one another are chosen from:
- hydrogen;
- (C₁-C₁₀)alkyl;
- (C₁-C₁₀)alkoxy;
- OH
- CN;
- CH₂CN;
- OCOR₈
- (C₁-C₆)fluoroalkyl;
- halogen;
- CON(R₈)₂;
- mono-, di- or tri-substituted phenyl
- mono-, di- or tri-substituted heteroaryl
the substituents being chosen from:
- hydrogen;
- halogen;
- (C₁-C₆)alkyl;
- (C₁-C₁₀)alkoxy;
- CN;
- CF₃
- N₃
- C(R₉)(R₁₀)-OH
- C(R₉)(R₁₀)-O-(C₁-C₄)alkyl;
- (C₁-C₆)fluoroalkyl;
• R₈ is chosen from:
- hydrogen;
- (C₁-C₆)alkyl;
- mono-, di- or tri-substituted phenyl, the substituents being chosen from hydrogen, halogen, (C₁-C₆)alkyl; (C₁-C₆)alkoxy; (C₁-C₆)alkylthio, CN or CF₃; and
- mono-, di- or tri-substituted benzyl, the substituents being chosen from hydrogen, halogen, (C₁-C₆)alkyl; (C₁-C₆)alkoxy; (C₁-C₆)alkylthio, CN or CF₃;
• or two R₈ groups form together with the nitrogen atom to which they are attached a ring having 5 to 7 atoms and optionally comprising a heteroatom chosen from O, S or NR₉;
• R₉ and R₁₀ independently of one another are chosen from:
- hydrogen; and
- (C₁-C₁₀)alkyl; or
form together with the nitrogen atom to which they are attached a ring having 3 to 7 carbon atoms and, if appropriate, a nitrogen atom;
**characterized in that** it comprises the following stages:
a) reaction of a compound of general formula II: 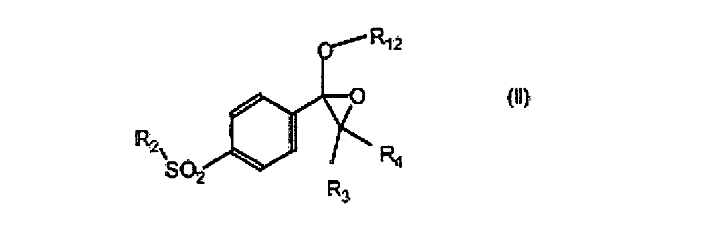 in which R₂, R₃, and R₄ are as defined previously and R₁₂ represents a C₁-C₆ alkyl group,
with an acid of general formula III: 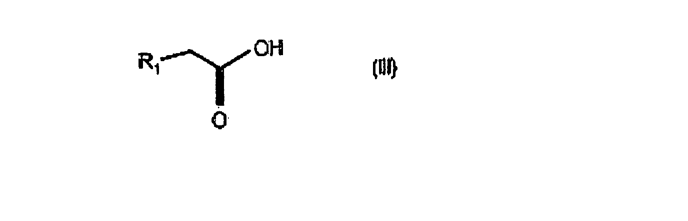 in which R₁ is as defined previously, in anhydrous medium, in order to form a compound of formula IV: 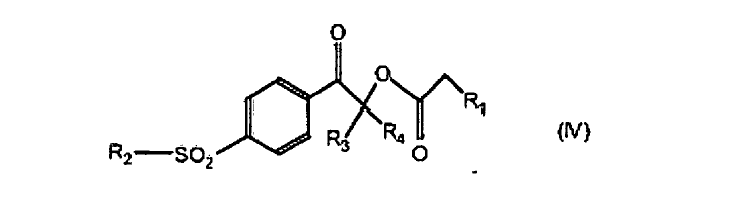 R₁ R₂, R₃, and R₄ being as defined above;
b) reaction of the compound of formula IV with a strong base in an aprotic solvent in order to obtain an intermediate cyclic compound of formula V: 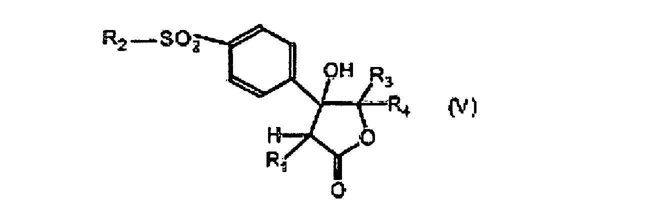 which, after elimination of a water molecule forms a compound of general formula I;
c) isolation of the compound of general formula I thus obtained.

2. Process according to claim 1, **characterized in that** the compound of general formula II is prepared by reaction of a compound of general formula VI: 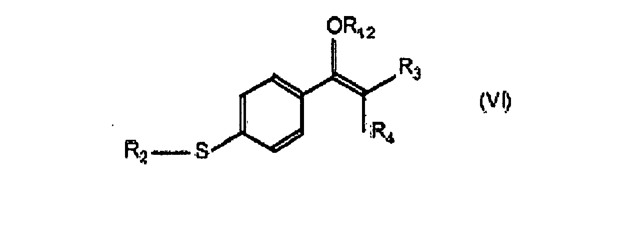 in which R₂, R₃, R₄ and R₁₂ are as defined in claim I, with an oxidizing agent.

3. Process according to claim 2, **characterized in that** the compound of general formula VI as defined in claim 2 is prepared by reaction of a compound of general formula VII: 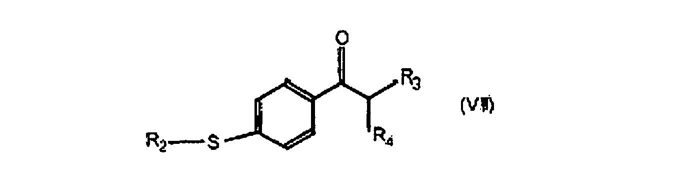 in which R₂, R₃, and R₄ are as defined in claim I, with an alcohol of general formula VIII:
HO R₁₂ (VIII)
R₁₂ being as defined above, in the presence of a catalytic quantity of acid and a dehydrating agent.

4. Process according to claim 3, **characterized in that** R₁₂ is methyl and the compound of general formula VIII is methanol.

5. Process for the preparation of compounds of general formula I as defined in claim 1, **characterized in that** it comprises the following stages:
(1) reaction of a compound of general formula (IX): 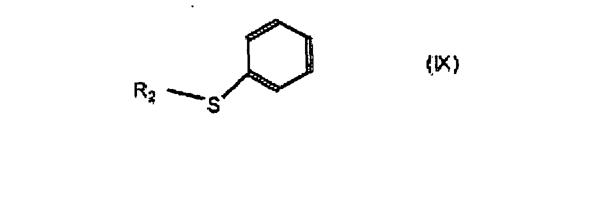 in which R₂ is as defined in claim 1, in an inert solvent in the presence of a Lewis acid with a compound of general formula X: 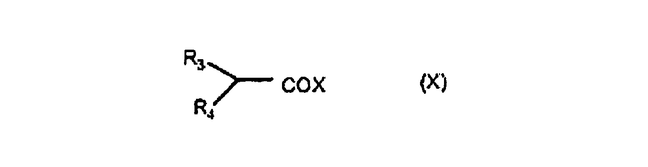 in which X is a leaving group, preferably a chlorine atom in order to form a compound of general formula VII: 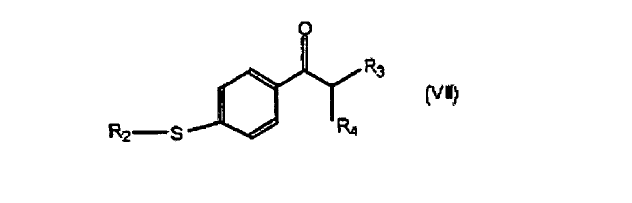 in which R₂, R₃, and R₄ are as defined in claim I,
(2) reaction of the compound of general formula VII with an alcohol of general formula VIII:
R₁₂-OH (VIII)
in which R₁₂ represents a (C₁-C₆)alkyl group in order to form a compound of general formula VI: 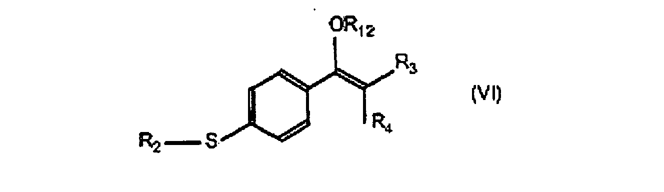 in which R₂, R₃, R₄ and R₁₂ are as defined previously,
(3) reaction of the compound of general formula VI with an oxidizing agent in order to obtain a compound of general formula II: 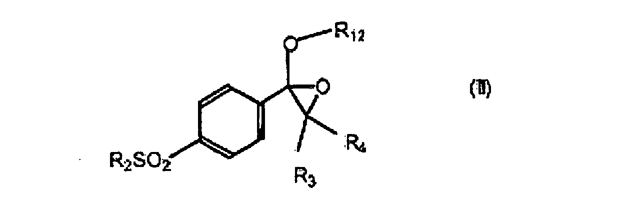 in which R₂, R₃, R₄ and R₁₂ are as defined previously,
(4) reaction of the compound of general formula II as defined in Stage (3)
with an acid of general formula III: 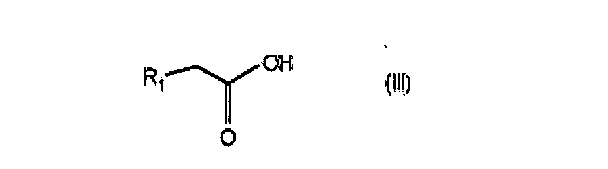 in which R₁ is as defined previously, in an anhydrous medium, in order to form a compound of formula IV: 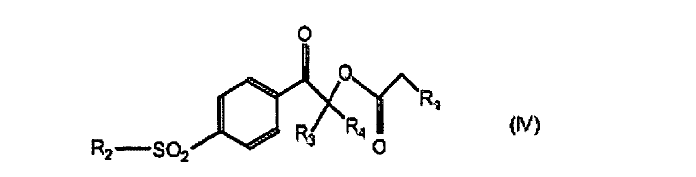 R₁, R₂, R₃ and R₄ being as defined above,
(5) reaction of the compound of formula IV with a strong base in an aprotic solvent in order to obtain an intermediate cyclic compound of formula V: 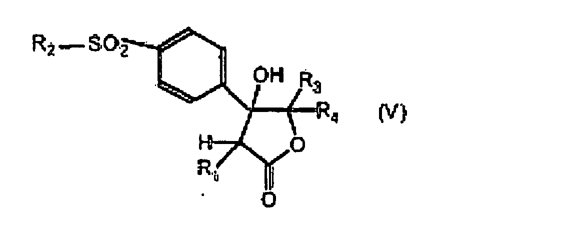 which, after elimination of a water molecule, forms a compound of general formula 1;
(6) isolation of the compound of general formula I thus obtained.

6. Process according to claim 1 for the preparation of a compound of general formula la: 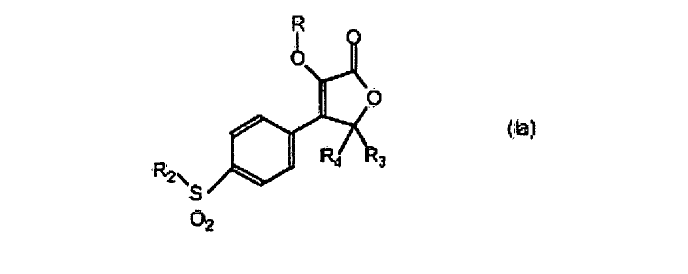 in which R represents an R₅ group as defined in claim 1 and R₂, R₃, and R₄ are as defined in claim 1, **characterized in that** it comprises the following stages:
a) reaction of a compound of general formula II: 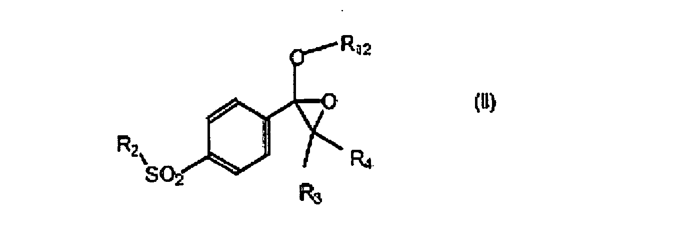 in which R₂, R₃, and R₄ are as defined previously and R₁₂ represents a C₁-C₆ alkyl group,
with an acid of general formula IIIa: 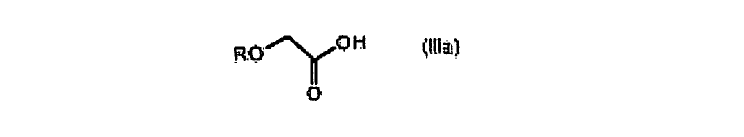 in which R is as defined above, in anhydrous medium in order to form a compound of general formula IVa: 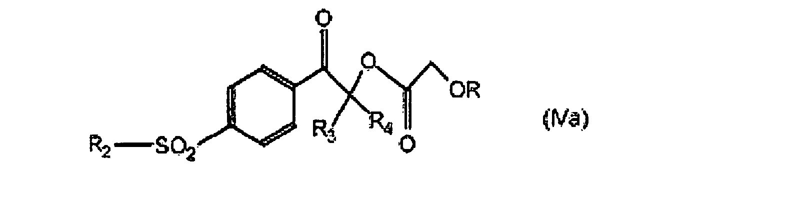 in which R, R₂, R₃, and R₄ are as defined previously.
b) reaction of the compound of formula IVa with a strong base in an aprotic solvent in order to obtain an intermediate cyclic compound of formula Va: 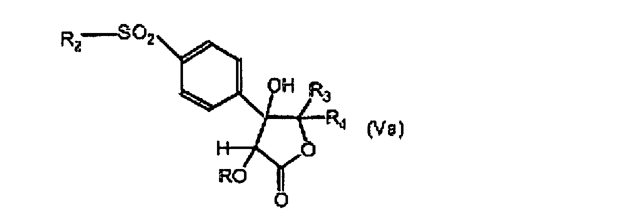 which, after elimination of a water molecule forms a compound of general formula la;
c) isolation of the compound of formula I thus obtained.

7. Process according to claim 1 for the preparation of a compound of general formula Ib: 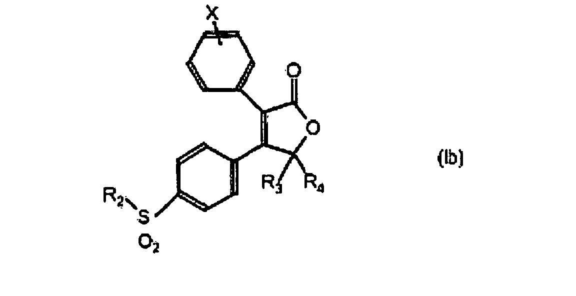 in which R₂ is as defined in claim 1 and X is chosen from:
- hydrogen;
- halogen;
- (C₁-C₃)alkoxy;
- CN;
- (C₁-C₃)fluoroalkyl;
- (C₁-C₃)alkyl;
- -COOH;
**characterized in that** it comprises the following stages:
a) reaction of the compound of general formula II 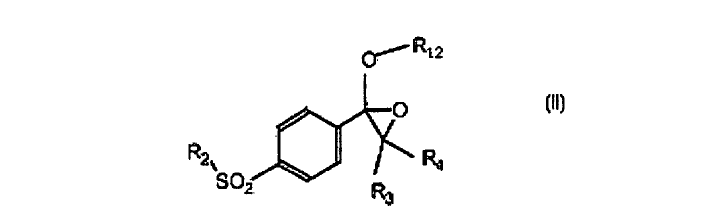 in which R₂, R₃, and R₄ are as defined previously and R₁₂ represents a (C₁-C₆)alkyl group,
with an acid of general formula III b): 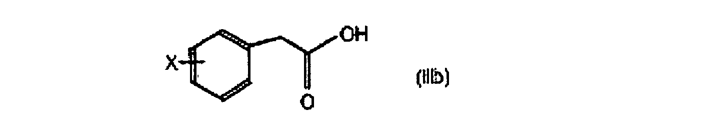 in which X is as defined previously, in anhydrous medium in order to form a compound of general formula IV b): 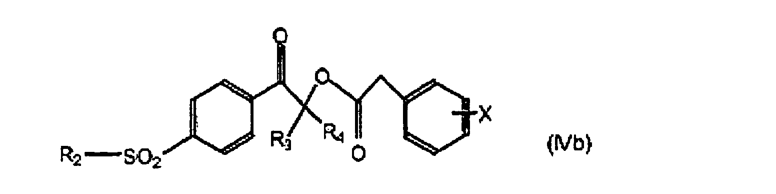 R₂, R₃, and R₄ being as defined above;
b) reaction of the compound of formula IVb with a strong base in an aprotic solvent in order to obtain an intermediate cyclic compound of formula Vb: 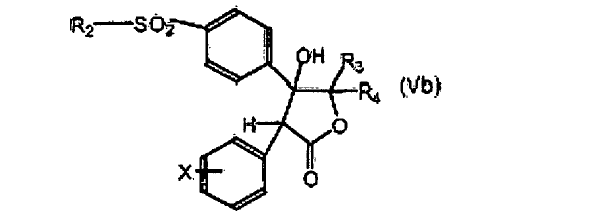 which, after elimination of a water molecule forms a compound of general formula Ib;
c) isolation of the compound of formula Ib thus obtained.

8. Process for the preparation of the compound of formula I: 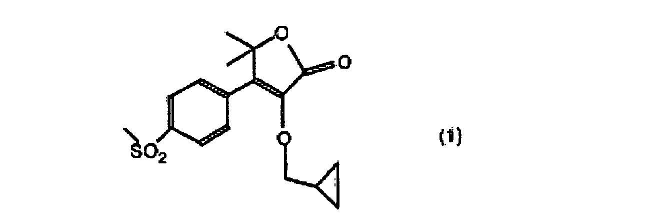 comprising the following stages:
- reaction of thioanisole in the presence of a Lewis acid with isobutyryl chloride in order to obtain the compound of formula (2): 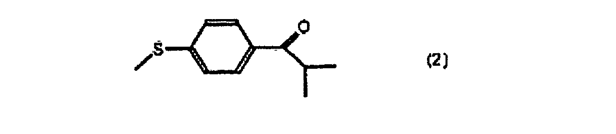
- reaction of the compound of formula (2) with methanol in the presence of para-toluene sulphonic acid and methyl orthoformate in order to form the compound of formula (3): 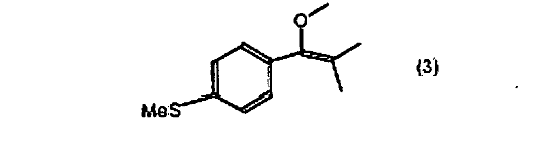
- reaction of compound (3) with dimethyldioxirane in order to form the compound of formula (4): 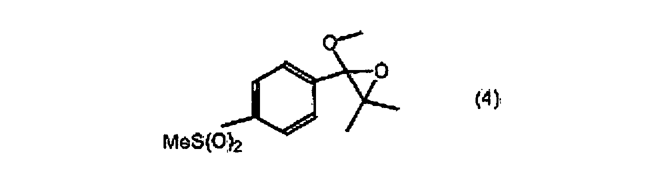
- reaction of compound (4) with cyclopropylmethyloxy acetic acid in an anhydrous solvent in order to obtain compound (5): 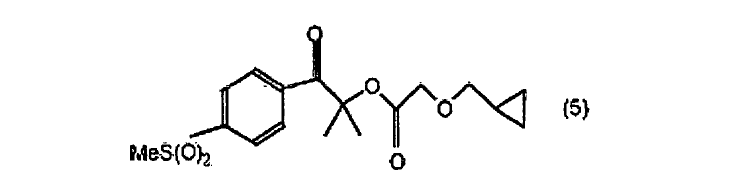
- reaction of compound (5) in an aprotic solvent with a strong base in order to obtain an intermediate cyclic compound (6): 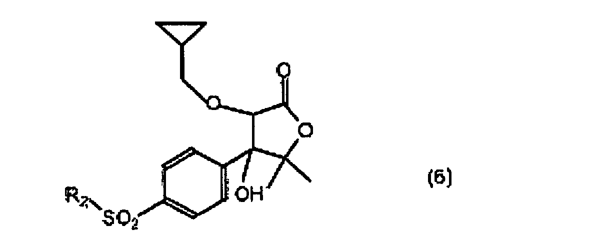 which, after dehydration in the presence of a dehydrating agent forms compound (1).

9. Process according to claim 2 or 5, **characterized in that** the oxidizing agent is chosen from the organic peracids, and the dioxiranes.

10. Process according to claim 2 or 5, **characterized in that** the temperature of the oxidation reaction is comprised between -40° and 30°C.

11. Process according to claim 2 or 5, **characterized in that** the oxidizing agent is used in excess relative to the compound of general formula II.

12. Process according to claim 3, **characterized in that** the acid is chosen from the sulphonic acids and the mineral acids.

13. Process according to claim 3, **characterized in that** a C₁-C₆ alkyl orthoformate is used as dehydrating agent.

14. Process according to claim 3 or 5, **characterized in that** the reaction is carried out in an excess of alcohol of general formula VIII, serving as reagent solvent.

15. Compound of general formula VI: 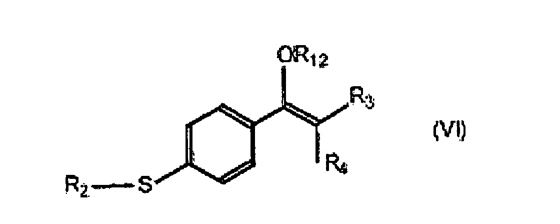 in which
• R₂ represents a (C₁-C₆)alkyl group;
• R₃ and R₄ represent independently of one another a hydrogen atom or a CHR₆R₇ group in which R₆ and R₇ independently of one another are chosen from:
- hydrogen;
- (C₁-C₁₀)alkyl;
- (C₁-C₁₀)alkoxy;
- OH
- CN;
- CH₂CN;
- OCOR₈
- (C₁-C₆)fluoroalkyl;
- halogen;
- CON(R₈)₂;
- mono-, di- or tri-substituted phenyl
- mono-, di- or tri-substituted heteroaryl
the substituents being chosen from:
- hydrogen;
- halogen;
- (C₁-C₆)alkyl;
- (C₁-C₁₀)alkoxy;
- CN;
- CF₃
- N₃
- C(R₉)(R₁₀)-OH
- C(R₉)(R₁₀)-O-(C₁-C₄)alkyl;
- (C₁-C₆)fluoroalkyl;
• R₈ is chosen from:
- hydrogen;
- (C₁-C₆)alkyl;
- mono-, di- or tri-substituted phenyl, the substituents being chosen from hydrogen, halogen, (C₁-C₆)alkyl; (C₁-C₆)alkoxy; (C₁-C₆)alkylthio, CN or CF₃; and
- mono-, di- or tri-substituted benzyl, the substituents being chosen from hydrogen, halogen, (C₁-C₆)alkyl; (C₁-C₆)alkoxy; (C₁-C₆)alkylthio, CN or CF₃;
• or two R₈ groups form together with the nitrogen atom to which they are attached a ring having 5 to 7 atoms and optionally comprising a heteroatom chosen from O, S or NR₉;
• R₉ and R₁₀ independently of one another are chosen from:
- hydrogen; and
- (C₁-C₁₀)alkyl; or
form together with the nitrogen atom to which they are attached a ring having 3 to 7 carbon atoms and, if appropriate, a nitrogen atom;
and R₁₂ represents a C₁-C₆ alkyl group.

16. Compound of formula 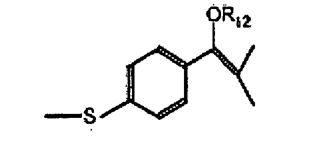 in which R₁₂ is as defined in claim 15, in particular, methyl.
